Europäisches Patentamt

European Patent Office

Office européen des brevets

(19)

(11) Veröffentlichungsnummer: **0 016 250**
**B1**

(12) # EUROPÄISCHE PATENTSCHRIFT

(45) Veröffentlichungstag der Patentschrift:
**12.10.83**

(51) Int. Cl.³: **A 61 B 5/08,** G 06 F 15/42

(21) Anmeldenummer: **79104440.7**

(22) Anmeldetag: **12.11.79**

(54) **Atemtrainingssystem mit Patientenrückkopplung.**

(30) Priorität: **13.11.78 US 959911**

(43) Veröffentlichungstag der Anmeldung:
**01.10.80 Patentblatt 80/20**

(45) Bekanntmachung des Hinweises auf die Patenterteilung:
**12.10.83 Patentblatt 83/41**

(84) Benannte Vertragsstaaten:
**DE FR GB IT NL SE**

(56) Entgegenhaltungen:
**DE-A-2 622 128**
**DE-A-2 749 160**
**US-A-2 333 791**
**US-A-3 621 835**
**US-A-3 754 546**
**US-A-3 818 901**
**US-A-3 822 699**
**US-A-3 922 525**
**US-A-3 977 394**
**US-A-4 025 070**
**US-A-4 037 836**
**US-A-4 078 554**
**US-A-4 114 607**
**US-A-4 114 608**

(73) Patentinhaber: **C.R. Bard, Inc., 731 Central Avenue,
Murray Hill, New Jersey 07974 (US)**

(72) Erfinder: **Elson, Edward E., 4346 Claytor Circle, Anaheim,
California 92806 (US)**

(74) Vertreter: **Görtz, Helmut, Dipl.-Ing. et al,
Schneckenhofstrasse 27, D-6000 Frankfurt am Main (DE)**

ACTORUM AG

Atemtrainingssystem mit Patientenrückkopplung

Die Erfindung betrifft ein Atemtrainingssystem mit Patientenrückkopplung, bestehend aus einem Atemstrommesser mit einer Grundplatte und einem aufrecht auf dieser angeordneten Geräteteil, welcher eine Ansaugluftmesskammer mit einem darin beweglichen Indikatorkörper aufweist, der sich in eine bestimmte Position bewegt, wenn der Ansaugluftstrom eine bestimmte, mittels eines Atemstromreglers einstellbare Durchflussmenge erreicht oder überschreitet.

Die Verwendung von Atemtrainingsgeräten zur Anregung eines Patienten, nach der Operation seine Lungen vollständig zu füllen, hat sich als erfolgreich erwiesen, um den Auftritt von Atemschwierigkeiten nach einer Krankheit zu vermindern.

Die gegenwärtig verfügbaren Atemtrainingsgeräte lassen sich in zwei Kategorien unterteilen, nämlich in disponierbare und permanente Geräte mit disponierbaren Elementen. Die disponierbaren Einheiten werden dem Patienten während seines Aufenthaltes im Krankenhaus überlassen, und er wird ermuntert, über sie einzuatmen, um seine Lungen vollständig aufzublasen und die vor der Operation verfügbare Lungenkapazität allmählich wieder zu erreichen. Einem Patienten wird beispielsweise verordnet, zehnmal in einer Stunde tief zu inhalieren, wobei dies mit allmählich steigendem eingeatmetem Volumen und Luftströmen erfolgt, bis seine Leistungsfähigkeit mindestens die Leistungsfähigkeit vor der Operation wieder erreicht hat.

Die permanenten bzw. wiederverwendbaren Einheiten sind verhältnismässig teuer, und sie enthalten ein wegwerfbares Element, das ein Luftstromfühler oder ein Volumenmessgefäss sein kann und zu jedem Zeitpunkt nur bei einem einzigen Patienten benutzt wird. Wenn eine Behandlung für einen Patienten vorgesehen ist, so wird der permanente Teil mit einem neuen wegwerfbaren Element für die Verwendung durch jeden einzelnen Patienten versehen. Eine Gebrauchseinschränkung der gegenwärtig verfügbaren permanenten Geräte liegt daher darin, dass sie den Tag über einem einzigen Patienten für die Benutzung überlassen werden müssen, wodurch ein Krankenhaus gezwungen ist, einen grossen Bestand von teuren Geräten zu unterhalten.

Anderseits sind preisgünstig hergestellte Atemstrommesser bekannt, die nur von einem bestimmten Patienten verwendet werden sollen und nach längerer Benutzung durch diesen Patienten weggeworfen werden. Mit diesem preiswerten, disponierbaren Atemstrommesser sind jedoch nur beschränkte Auswertungen eines Atemtrainingsprogramms möglich. Ein disponierbarer Atemstrommesser vom eingangs bezeichneten Typ, der in diese Gruppe fällt, ist in der DE-A1-2 749 160 beschrieben.

Dieser Atemstrommesser verfügt über eine Anordnung, um einen Patienten zu veranlassen, einen Luftstrom über einen Strömungsweg einzuatmen, der einen Durchflussregler oder -begrenzer aufweist. Die Inhalierung mit einem voreingestellten Durchfluss veranlasst einen sichtbaren Indikator, wie beispielsweise eine Kugel, in einem Rohr nach oben zu steigen. Ein Therapeut kann ein Programm von Atmungsübungen aufstellen, bei dem der Patient aufgefordert ist, die Kugel in dem Rohr nach oben anzuheben und sie dort für eine bestimmte Zeit zu halten. Der Durchflussregler kann so eingestellt werden, dass verschiedene minimale Durchflussmengen für die Anhebung der Kugel nach oben erforderlich sind. Vom Patienten ist anzustreben, den festgelegten Durchfluss zu erzielen und die Kugel im oberen Bereich des Rohres für eine festgelegte Zeit zu halten, wobei dies einige Male am Tag geschehen kann. Diese Art von Programm bietet eine bestimmte, minimal erforderliche Überwachung für Atmungsübungen.

Um ein solches Gerät zu betreiben, ist es für den Patienten oder den Therapeuten nämlich erforderlich, Aufzeichnungen mittels eines Bleistiftes oder Papieres und einer Stoppuhr zu machen. Dabei wird die Anzahl der durch den Patienten durchgeführten Atemanstrengungen vermerkt, bei denen er den Durchfluss für eine festgelegte Zeit erzielt, um die Zahl der erfolgreichen Versuche festzuhalten. Um das inhalierte Luftvolumen bei einem bestimmten Versuch festzustellen, muss die Zeit, in der die Kugel im oberen Rohrbereich der Durchflusskammer gehalten wird, gemessen werden, was beispielsweise durch eine Stoppuhr erfolgt. Kennt man den Durchfluss, der mittels des Regelventils eingestellt ist, so kann beispielsweise anhand eines Diagramms das Volumen der inhalierten Luft festgestellt werden, oder es kann der Durchfluss in Kubikzentimetern pro Sekunde mit der zugehörigen Haltezeit multipliziert werden.

Die Verwendung solcher Geräte ist umständlich, da entweder der Patient mit einer Stoppuhr ausgerüstet werden muss, um seine eigenen Aufzeichnungen zu führen, oder ein Therapeut zur rechten Zeit vorhanden sein muss, um immer dann Aufzeichnungen vorzunehmen, wenn der Patient einen Versuch zur Anhebung der Kugel ausführt.

Die Anwesenheit eines Therapeuten kann ebenfalls erforderlich sein, um sicherzustellen, dass der Atemstrommesser in geeigneter Weise benutzt wird. Wenn beispielsweise ein Patient im Bett liegt und den Atemstrommesser anwendet, kann er das Gerät auf seiner Bettdecke oder auf seinem Bauch plazieren. Höchstwahrscheinlich befindet sich hierbei der Atemstrommesser nicht genau in der Vertikalen. Exakte Messungen des Luftstromes sind aber nur dann möglich, wenn der Atemstrommesser vertikal ausgerichtet ist. Je weiter dessen Lage von der Vertikalen abweicht, um so ungenauer sind die Messungen bezüglich der zur Anhebung der Kugel erforderlichen Anstrengungen.

Anderseits sind bereits aufwendige Systeme für die Vorgabe und Auswertung von Atemtrainingsprogrammen bekanntgeworden, bei denen es sich jedoch um verhältnismässig teure Steuer- und Messapparaturen handelt, die weit davon entfernt sind, für ein kompaktes Wegwerfgerät geeignet zu sein, wie es dasjenige gemäss der DE-A1-2 749 160 darstellt. Ein solches vielseitiges Atemtrainings- und -beobachtungssystem mit Bio-Rückkopplung ist beispielsweise aus der DE-A1-2 622 128 bekannt. Dieses bekannte Atemtrainingssystem beruht auf dem Grundprinzip, dass die üblicherweise vom Patienten unwillkürlich ausgeübte Atemfunktion durch geeignete Mittel messend aufgenommen und im Vergleich mit anzustrebenden Idealwerten auf einem Bildschirmmonitor sichtbar angezeigt wird, so dass der Patient mit der eigenen Leistung konfrontiert werden kann. Das bekannte Trainingssystem empfängt seinen Input von einem patientenseitig angeordneten Atemstrommesser irgendeiner bekannten Bauart. Das von diesem Atemstrommesser erhaltene Input-Signal wird in Relation zu einer Zeitbasis integriert und das integrierte Signal zur Realzeit mit vorgegebenen gewünschten Werten auf der Zeitbasis entsprechend einem vorgegebenen Kurvenverlauf verglichen, wobei der Einatmungsbeginn zur Auslösung einer idealen Zyklusanzeige für Atemvolumina ermittelt wird.

Das bekannte Trainingssystem verfügt ferner über voreinstellbare Speicher zur Speicherung von Daten entsprechend dem gewünschten Minimalvolumen der vom Patienten in einem einzigen Atemzug zu atmenden Luft. Ferner weist das Trainingssystem eine Volumenmesseinrichtung auf, die aus dem vom Atemstrommesser ausgesandten Analogsignal zuerst den Zeitpunkt zum Beginn des Messzyklus bestimmt, dann Zeitgeber sowie einen Integrator zur Bestimmung des geatmeten Volumens in Gang setzt und das Ergebnis der Integration in einem Speicher ablegt. Dieses Ergebnis wird mit den Vorgabewerten in den Speichern verglichen. Das bekannte Trainingssystem sieht darüber hinaus Anreizvorrichtungen vor, die es gestatten, entweder zu vorwählbaren Zeiten während des vorgegebenen Atemzyklus und/oder bei vorwählbaren Abweichungen der Effektiv- von der Idealkurve durch verschiedenfarbige Lichter oder akkustische Summer repräsentierte Anzeigen zum Anreiz des Patienten zu aktivieren.

Mit diesem System lassen sich daher unterschiedlichste Trainingsprogramme durchführen.

Zwar ist dieses bekannte Atemtrainingssystem sehr vielseitig, es lässt sich aber kaum als einfach handhabbares und kompaktes Gebrauchsgerät von einem Einsatzort zum anderen verbringen. Ausserdem ist für seinen Einsatz ein Atemstrommesser erforderlich, der seinerseits zumindest über einen Messwandler verfügt, um das für das Trainingssystem erforderliche Input-Signal zu erzeugen. Derartige Einrichtungen sind aber für einen wegwerfbaren Atemstrommesser bereits meist schon zu aufwendig.

Ein weiteres, weniger aufwendiges Gesamtgerät zur Durchführung und Überwachung eines einfachen Trainingsprogramms ist beispielsweise der aus der US-A-3 754 546 bekannte Atemstrommesser, der aus einem in einem Zylinder mit vorbestimmter Leckrate beweglichen Kolben besteht. Die Grösse des Atemstromes bestimmt die senkrechte Position dieses Kolbens und damit eines mit ihm verbundenen, als Indikator dienenden Stabes, der in einer senkrechten Hülse gleitet. Bei Erreichen eines bestimmten Wertes des Atemstromes schliesst das obere Ende dieses Stabes einen Schalter und betätigt einen Stromkreis. Der Atemstromwiderstand bei diesem Atemstrommesser ist durch Regelung der Leckrate des Kolbens im Zylinder einstellbar. Aber auch dieser bekannte Atemstrommesser besitzt den Nachteil, dass er keine Trennung von Atemstrommesser und Auswerteinheit gestattet, weil der Indikatorkörper über einen Berührungsschalter arbeitet und der Atemstrommesser so über elektrische Mittel mit der Auswerteinheit verbunden ist, weswegen dieses bekannte Gerät auch dafür ausgelegt ist, Atemstrommesser und Auswerteinheit in ein und demselben Gerät aufzunehmen.

Durch die Erfindung wird aber angestrebt, den disponierbaren Atemstrommesser schaltungsmässig vollständig unabhängig von der Auswerteinheit zu belassen, um eine Trennbarkeit zwischen dem disponierbaren Teil des Gerätes und dem immer wieder einsetzbaren Auswertteil des Gerätes herbeiführen zu können. Die Aufgabe der Erfindung ist daher darin zu sehen, einen für den Gebrauch durch nur einen Patienten mit einfachsten Mitteln, aber ohne weiteres in sich selbst funktionsfähigen Atemstrommesser, der nach Gebrauch durch den betreffenden Patienten weggeworfen werden kann, auf einfache Weise mit Mitteln zur Erfassung und Auswertung der Atemübungsvorgänge zu versehen, welche einen höheren gerätetechnischen Aufwand erfordern, ohne dass irgendwelche schaltungsmässigen Verbindungen zwischen dem Atemstrommesser und der Auswerteinheit hergestellt werden müssten, sondern eine Verbindung sich allein auf das formschlüssige Zusammenfügen der beiden Geräteteile beschränkt. Dabei soll in seiner speziellen Ausführung die Auswerteinheit auch so ausgebildet sein, dass sie überflüssigen Aufwand vermeidet und in kompakter Weise mit dem Atemstrommesser kombinierbar ist.

Ein Atemtrainingssystem des eingangs bezeichneten Typs ist erfindungsgemäss gekennzeichnet durch ein mit dem Atemstrommesser formschlüssig zu einem Gesamtgerät verbindbares Zusatzgerät mit einem berührungslos durch Aussenden und Empfangen einer Energiestrahlung arbeitenden Signalgeber, welcher ein Signal erzeugt, solange sich der Indikatorkörper in der bestimmten Position befindet, wobei das Zusatzgerät die signalverarbeitende Auswerteinrichtung enthält und über eine an ihm vorgesehene alphanumerische Anzeigeeinrichtung verfügt. Bei einer bevorzugten Ausführungsform besteht der Signalgeber aus der Kombination einer Lichtquelle mit einem Fotodetektor, in welchem Fall der Indikatorkörper natürlich lichtundurchlässig ist.

Die erfindungsgemässe Ausführung ermöglicht es, dem Patienten seinen Atemstrommesser für bestimmte Übungen und für seinen ausschliesslichen persönlichen Gebrauch ständig zu belassen, die kompakte transportable und auf einfachste Art und Weise mit allen Atemstrommessern des gleichen Typs verbindbare Auswerteinheit nacheinander verschiedenen Patienten zur jeweiligen Durchführung ihrer Trainingsprogramme zur Verfügung zu stellen, ohne dass ein Therapeut den Ablauf dieser Programme überwachen müsste.

Vorteilhafte Ausgestaltungen des Zusatzgerätes bezüglich der in ihm enthaltenen Auswerteinheit und der Anzeigeeinrichtungen sind in weiteren Unteransprüchen beansprucht.

Anhand der Figuren der beiliegenden Zeichnung sei im folgenden ein Ausführungsbeispiel der vorliegenden Erfindung näher beschrieben. Es zeigen:

Fig. 1 eine Vorderansicht des erfindungsgemässen Gerätes.

Fig. 2 eine teilweise geschnittene Seitenansicht des Gerätes entlang der Linie 2–2 in Fig. 1.

Fig. 3 eine Schnittansicht entlang der Linie 3–3 in Fig. 2.

Fig. 4 eine Schnittansicht entlang der Linie 4–4 in Fig. 2.

Fig. 5 eine Ansicht teilweise im Schnitt entlang der Linie 5–5 in Fig. 2.

Fig. 6 ein Flussdiagramm für den Dateneingabe-Operationsmodus der Erfindung.

Fig. 7 ein Flussdiagramm für den Patienten-Operationsmodus gemäss der Erfindung.

Fig. 8 ein Blockdiagramm des erfindungsgemässen Gerätes.

Fig. 9 ein detailliertes Schaltkreisdiagramm eines Teiles des Blockdiagramms gemäss Fig. 8.

Fig. 10 ein detailliertes Schaltkreisdiagramm des restlichen Blockdiagramms gemäss Fig. 8.

Fig. 11 ein Funktions-Blockdiagramm für den Betrieb des Mikroprozessors in Fig. 10.

Fig. 12 ein Beispiel für die Anzeige-Ausgabe.

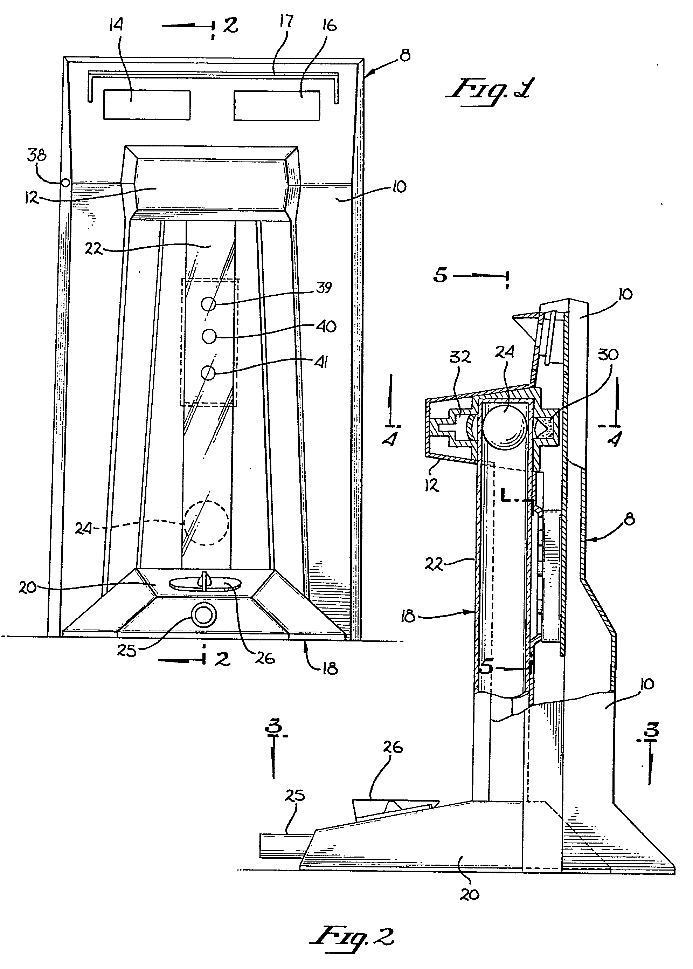

Die Fig. 1 bis 5 zeigen das Atemtrainingssystem, bestehend aus dem Atemstrommesser und dem Zusatzgehäuse mit der Auswerteinrichtung. Der Einfachheit halber sei das Atemtrainingssystem nachstehend als Gerät bezeichnet. Das vorliegende Gerät umfasst ein Zusatzgerät 8, das insbesondere in Fig. 1 dargestellt ist. Das Zusatzgerät umfasst in erster Linie zwei Teile. Der erste Teil ist der Hauptkörper 10, und der zweite Teil ist das Detektorgehäuse 12, das sich von dem oberen Vorderteil des Hauptkörpers 10 weg erstreckt. Unmittelbar oberhalb des Detektorgehäuses befindet sich eine Anzeigeeinrichtung 160 mit einer linken Anzeigeeinheit 14 und einer rechten Anzeigeeinheit 16. Damit die innerhalb dieser Einrichtungen angezeigten Daten leichter lesbar sind, ist ein Lichtschirm 17 vorgesehen, der sich von dem Hauptkörper 10 nach aussen erstreckt und der das auf die Einrichtungen gerichtete Licht abschirmt. Das Detektorgehäuse 12 ist mit einer sich nach unten erstreckenden Ausnehmung versehen, die für einen geeigneten Eingriff mit einem Atmungsmesser 18 ausgelegt ist, wodurch das Zusatzgerät 8 lösbar in den Atemstrommesser 18 eingesetzt werden kann. Der Atemstrommesser umfasst eine Grundplatte 20, eine sich vertikal erstreckende Ansaugluftmesskammer 22 und innerhalb der Kammer einen beweglichen Indikatorkörper 24, der frei bis zu dem oberen Ende der Kammer 22 infolge eines Luftstromes durch die Kammer ansteigen kann. Der Indikatorkörper 24 kann aus einer Kugel oder einer anderen Einrichtung bestehen, die für die Zwecke des bevorzugten Ausführungsbeispieles des vorliegenden Atemtrainingssystems für Licht nicht durchlässig ist. Durch Inhalierung durch die Mündung 25 kann ein Patient einen Luftstrom durch die Kammer 22 erzeugen, der seinerseits den Indikatorkörper 24 zum Ansteigen innerhalb der Kammer 22 veranlasst. Wenn die Grösse des durch den Patienten inhalierten Luftstromes gleich dem Luftstrom ist, der durch den Atemstromregler 26 eingestellt ist oder diesen Luftstrom übertrifft, so wird der Indikator 24 bis zum oberen Ende der Kammer 22 ansteigen, wodurch angezeigt wird, dass der Patient eine Atemstärke erreicht hat, die wenigstens der Einstellung des Ventils 26 enspricht.

Gemäss Fig. 2 beinhaltet das Detektorgehäuse 12 eine Lichtquelle 30 und einen Fotodetektor 32. Die Lichtquelle und der Fotodetektor stehen miteinander mittels eines Lichtstrahles in Verbindung, der von der Lichtquelle 30 zu dem Fotodetektor 32 verläuft. Dies ist möglich, obgleich das Zusatzgerät 8 mit dem Atemstrommesser 18 verbunden ist, da die Kammer 22 des Atemstrommessers für die Lichtstrahlung durchlässig ist, welche von der Lichtquelle 30 ausgesendet wird. Es liegt dem Fachmann auf der Hand, dass anstelle der Lichtquelle 30 und des Fotodetektors 32 ein Näherungsdetektor, wie beispielsweise ein Magnetfühler, verwendet werden kann. Durch die vorliegende Erfindung sollen jede Art von Energiequelle und Detektor umfasst werden, die einen Hinweis dafür liefern, dass eine Indikatoreinrichtung an einem bestimmten Ort anwesend oder abwesend ist, wobei dieser Ort in der Kammer 22 des Atmungsmessers liegt. Es sei ferner darauf verwiesen, dass mehrere solcher Detektoren an verschiedenen Orten entlang der Länge der Kammer 22 verwendet werden können, um das Vorliegen des Indikatorkörpers an verschiedenen Orten beim Anstieg des Indikators in der Kammer festzustellen. Anstelle des Lichtstrahles kann auch ein Schaltstrahl, ein radiofrequenter Strahl oder irgendein anderer geeigneter Energiestrahl verwendet werden.

Wenn die Atmung des Patienten durch die Mündung 25 dem minimalen, am Atemstromregler 26 eingestellten Durchfluss entspricht, so veranlasst der Luftstrom durch die Kammer 22 den Indikatorkörper 24 zum Anstieg bis zum oberen Ende der Kammer 22, wie dies in Fig. 2 dargestellt ist. Da der Indikatorkörper 24 so gewählt ist, dass er für die von der Lichtquelle 30 ausgesandte Energie nicht transparent ist, wird durch die Gegenwart des Indikators 24 der Lichtstrahl unterbrochen, der von der Lichtquelle 30 zu dem Fotodetektor 32 fliesst, was zu einer Unterbrechung der Verbindung zwischen Energiequelle und Fotodetektor führt. Diese Verbindungsunterbrechung ist von besonderer Bedeutung im Hinblick auf die vorliegende Erfindung, was noch näher zu erläutern sein wird.

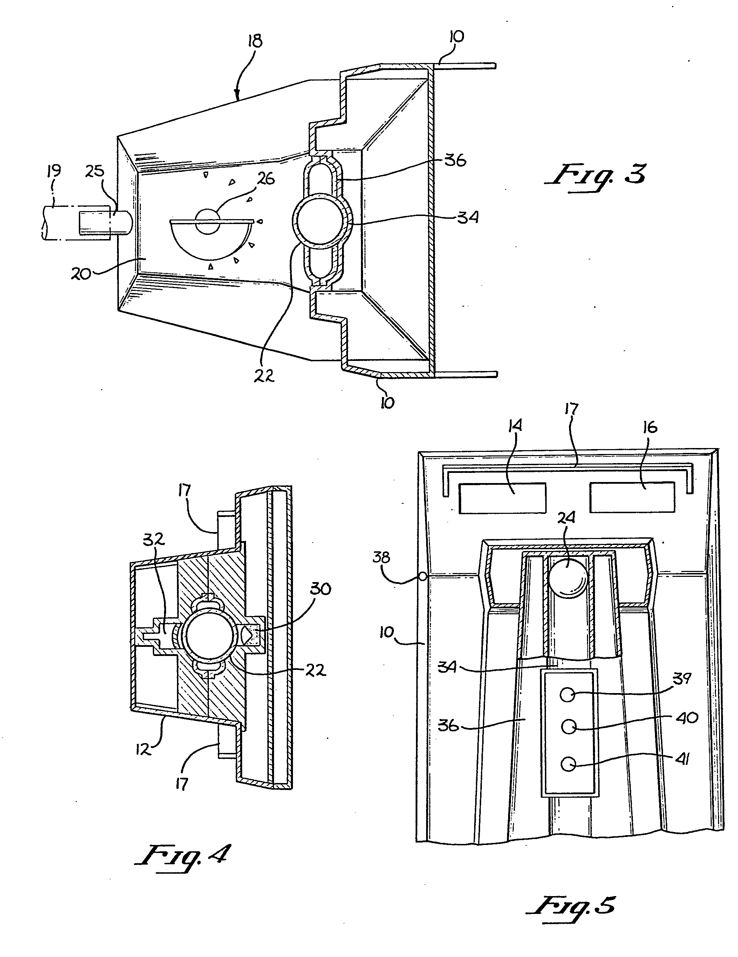

Um die geeignete Anpassung des Zusatzgerätes 8 an den Atemstrommesser 18 zu erleichtern, ist der Grundkörper 10 des Zusatzgerätes mit Ausnehmungen 34 und 36 versehen, die so gestaltet sind, dass sie sich der Kammer 22 anpassen. Dieses Merkmal ist besonders deutlich in Fig. 3 veranschaulicht. Diese Ausnehmungen 34 und 36 erstrecken sich längs der vertikalen Länge des Zusatzgerätes 8 entsprechend der vertikalen Profilierung der Kammer 22. Fig. 3 zeigt auch den Atemstromregler 26. In dem speziell dargestellten Atemstrommesser bildet der Atemstromregler 26 lediglich eine veränderliche Ventilöffnung. Die Öffnung liefert geeichte Durchflussmengen entsprechend der speziellen Grösse der Öffnung, wenn der Zeiger des Reglers auf eine bestimmte Eichung oder Scheibeneinstellung verweist. Die Eichung erfolgt allgemein in Kubikzentimetern pro Sekunde und zeigt den Durchfluss an, der erzielt werden muss, um den Indikatorkörper an die Spitze der Kammer 22 anzuheben. Durch Einatmen durch ein langes flexibles Rohr 19 ruft der Patient einen Luftstrom durch die Mündung 25 und die Kammer 22 hervor. Wenn der Luftstrom die Einstellung des Atemstromreglers 26 erreicht oder übersteigt, so wird der Indikatorkörper 24 bis zur Spitze der Kammer 22 angehoben.

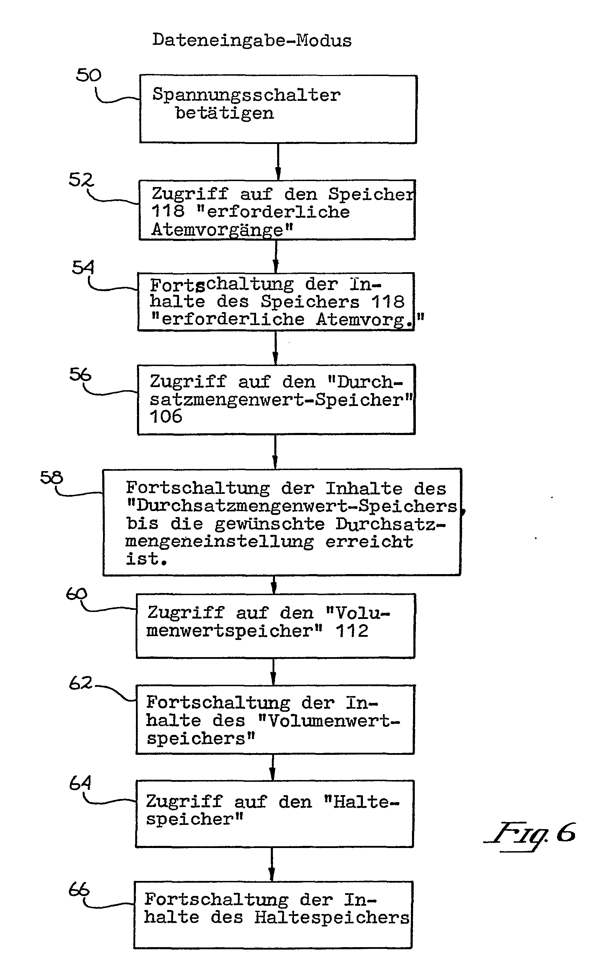

Gemäss Fig. 4 erstreckt sich die Spitze der Kammer 22 zwischen der Lichtquelle 30 und dem Fotodetektor 32. Wenn der Indikator 24 bis zu der Spitze der Kammer 22 angehoben wird, so unterbricht der Indikator die von der Lichtquelle 30 zu dem Fotodetektor 32 ausgesandte Lichtenergie.

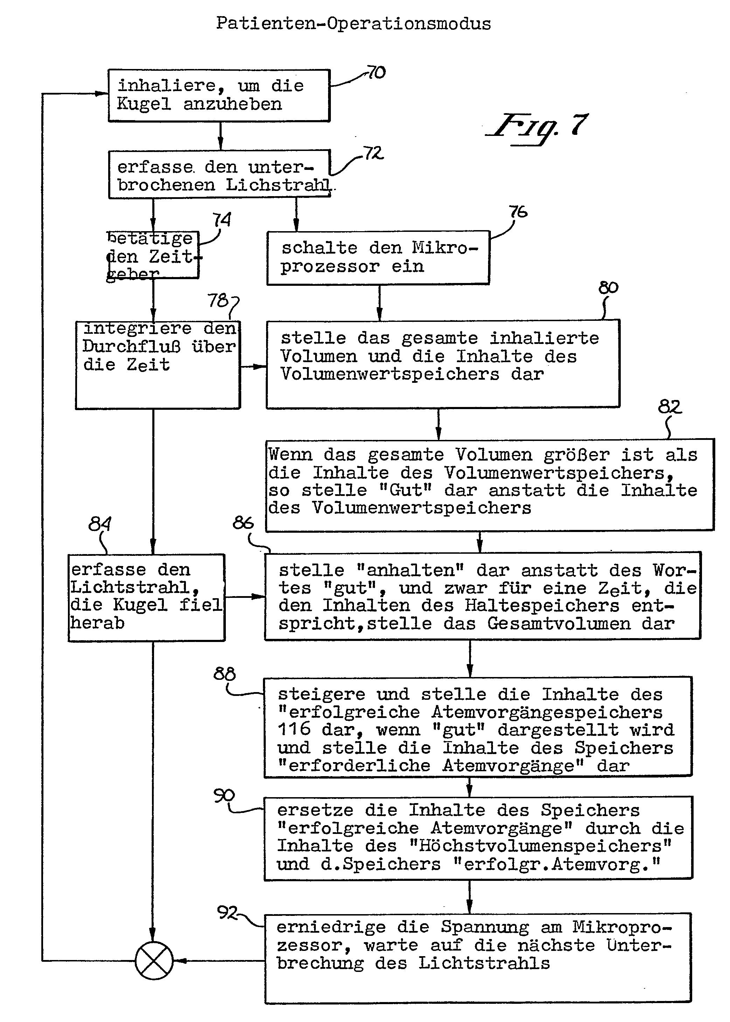

Wie zuvor erläutert, ist die Verwendung des vorliegenden Zusatzgerätes im Zusammenhang mit einem Atemstrommesser beabsichtigt. Ein Atemstrommesser ist ein Gerät, das benutzt wird, um sicherzustellen, dass einem Patienten nach einer Operation ein Anreiz für die Übung seiner Lungen gegeben wird, um die Möglichkeit von Atemkomplikationen zu vermindern. Im allgemeinen Fall stellt ein Therapeut in einem Krankenhaus ein solches Übungsprogramm auf, dem der Patient zu folgen hat. Ein solches Programm kann von dem Patienten fordern, dass er eine bestimmte minimale Inhalierung für eine festgelegte Zeit erzielt, so dass der Patient ein vorbestimmtes minimales Luftvolumen aufnimmt. Als Teil des Programms kann von dem Patienten gefordert werden, dass er dieses Volumen mit einem bestimmten minimalen Luftstrom inhaliert, wobei dies mehrere Male im Verlaufe einer Stunde oder einer anderen vorgeschriebenen Zeitspanne zu geschehen hat. An einen derartigen Patienten kann ebenfalls die Forderung gestellt werden, dass er das inhalierte Luftvolumen in seinen Lungen für eine festgelegte Zeitspanne hält. Es ist somit ersichtlich, dass es eine Anzahl vorbestimmter Werte gibt, die von einiger Bedeutung bei der Definition des Programms der Atemübung sind, welcher der Patient zu folgen hat. Insbesondere umfassen diese Parameter den minimal zu erzielenden Durchfluss, das minimal zu inhalierende Gesamtvolumen, die Zeitdauer, in der der Patient das inhalierte Volumen zu halten hat und die Anzahl der Atemübungen, bei der der Patient dieses vorbestimmte minimale Volumen inhalieren muss. Das vorliegende Zusatzgerät verfügt über eine Einrichtung zur Eingabe der Werte dieser Parameter in einen Mikroprozessor, der einen Teil des Zusatzgerätes bildet. Die besonderen Mittel, durch die solche Parameter in den Mikroprozessor eingegeben werden, umfassen den Speicher-Zugriffsschalter 38, der ebenfalls alleine benutzt wird, um die Daten in einem Speicher darzustellen, den Daten-Eingabeschalter 39, den Löschschalter 40 und den Ein/Aus-Schalter oder Spannungsschalter 41. Der Ort dieser Schalter ist in Fig. 5 dargestellt. Insbesondere können diese Schalter innerhalb der Ausnehmung 34 angeordnet sein. Es sei jedoch ausdrücklich darauf verwiesen, dass andere Orte mit gleichen Vorteilen in Frage kommen, wobei jeder Ort auf der Aussenfläche des Zusatzgerätes in Frage kommt, wie dies im Falle des Speicher-Zugriffschalters 38 der Fall ist. Wenn der Therapeut das Programm der Atemübung, das für den speziellen Patienten nach der Operation geeignet ist, einmal festgelegt hat, so werden die zuvor beschriebenen Parameter in den Mikroprozessor durch eine geeignete Aktivierung des zugeordneten Schalters eingegeben. Wenn das Gerät von dem Therapeuten betätigt wird, um die zuvor beschriebenen Parameter einzugeben, so sei dieser Betrieb als Daten-Eingabemodus bezeichnet. Dieser Operationsmodus ist schematisch in Fig. 6 veranschaulicht.

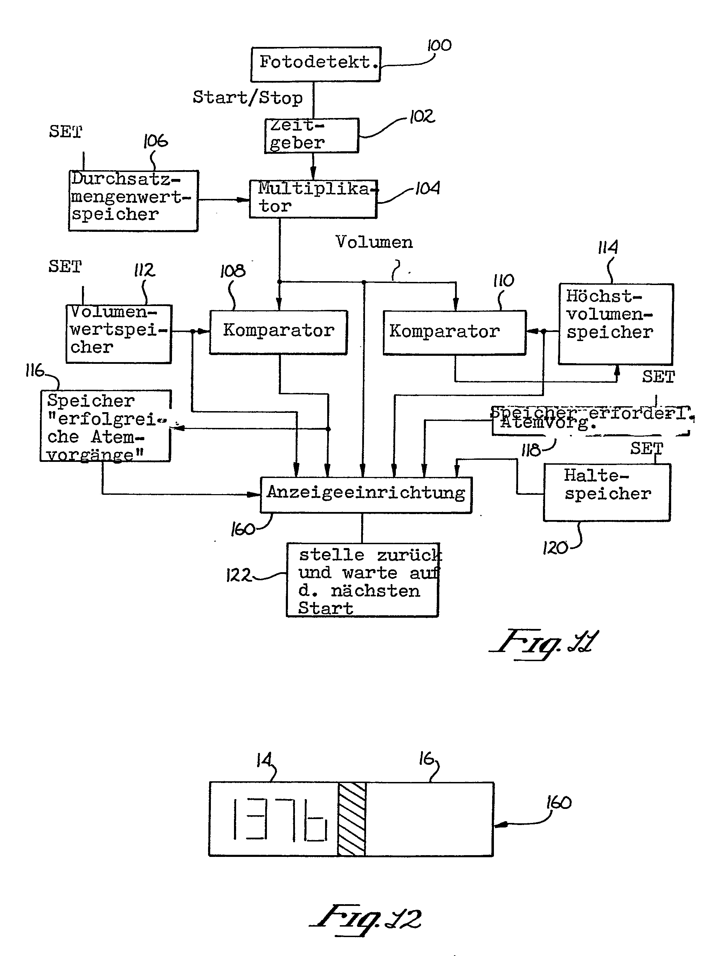

Das Zusatzgerät wird vorzugsweise durch eine Batterie betrieben und ist mit einem Ein/Aus-Schalter 41 versehen. Die Operationsfolge bei der Dateneingabe in den Mikroprozessor ist in den Schritten 50 bis 66 in Fig. 6 dargestellt. Im Schritt 50 muss der Therapeut den Spannungsschalter betätigen. Um Daten in den Mikroprozessor einzugeben, muss der Therapeut das geeignete Speicherregister wählen. Ein Zugang zu einem bestimmten Speicherregister wird durch Aktivierung des Speicher-Zugriffsschalters 38 gewonnen. Das Niederdrücken des Schalters 38 gestattet den Zugriff auf die verschiedenen Speicherregister in einer vorprogrammierten Reihenfolge. Das Niederdrücken des Schalters 38 gestattet beim ersten Mal den Zugriff auf den Speicher «erforderliche Atemvorgänge» 118 (siehe Fig. 11). Die in dem Speicher gespeicherten Daten werden in den Anzeigeeinheiten 14 und 16 dargestellt. Die nächste Betätigung des Schalters 38 gestattet den Zugriff auf den «Höchstvolumen-Speicher» 114. In gleicher Weise gestattet eine aufeinanderfolgende Betätigung des Schalters 38 den Zugriff auf den «Durchsatzmengenwert-Speicher» 106, den «Volumenwert-Speicher» 112 und den «Halte-Speicher» 120. Der Dateninhalt eines jeden speziellen Speichers kann durch Zugriff auf den speziellen Speicher in der zuvor beschriebenen Weise und durch Halten des Speicher-Zugriffsschalters 38 im niedergedrückten Zustand eingegeben werden, während der Daten-Eingabeschalter 39 betätigt ist. Durch Betätigung des Daten-Eingabeschalters 39 wird der Dateninhalt des speziellen Speichers, auf den Zugriff genommen wird, in einer vorbestimmten Reihenfolge erhöht. Wenn auf den Speicher «erforderliche Atemvorgänge» 118 Zugriff genommen wird, so wird durch eine aufeinanderfolgende Betätigung des Daten-Eingabeschalters 39 der Inhalt dieses Speichers 118 um Eins bei jeder Betätigung des Daten-Eingabeschalters 39 fortgeschaltet. Wenn auf «Durchsatzmengenwert-Speicher» 106 Zugriff genommen wird, so wird durch eine aufeinanderfolgende Betätigung des Daten-Eingabeschalters 39 der Inhalt dieses Speichers 106 in Schritten erhöht, die den Eichungen des Atemstromreglers 26 entsprechen. Wenn somit auf den Speicher 106 Zugriff genommen wird und der Speicher-Zugriffsschalter 38 im niedergedrückten Zustand gehalten wird, so wird durch die aufeinanderfolgende Betätigung des Daten-Eingabeschalters 39 der Inhalt des «Durchsatzmengenwert-Speichers» 106 erhöht. Dies setzt sich fort, bis der Wert von 1800 Kubikzentimeter pro Sekunde erreicht ist, zu welchem Zeitpunkt bei der nächsten Betätigung des Daten-Eingabeschalters 39 der Inhalt des Speichers 106 auf den Ausgangspunkt von 145 Kubikzentimeter pro Sekunde zurückgeführt wird. In gleicher Weise kann der Inhalt der verschiedenen anderen Speicher stufenweise durch aufeinanderfolgende Betätigung des Daten-Eingabeschalters 39 verändert werden, während der Speicher-Zugriffsschalter 38 im niedergedrückten Zustand gehalten wird.

Die Betriebsweise des Zusatzgerätes 8 in dem Daten-Eingabemodus durch den Therapeuten ist somit zweckdienlich in Fig. 6 veranschaulicht. Der Therapeut schaltet zunächst das Gerät im Schritt 50 ein. Der Therapeut betätigt sodann den Speicher «erforderliche Atemvorgänge» 118, was im Schritt 52 dargestellt ist. Dies geschieht durch Niederdrücken des Zugriffsschalters 38 und durch nachfolgendes Niederdrücken des Daten-Eingabeschalters 39. Während der Zugriffsschalter 38 im niedergedrückten Zustand gehalten wird, wird der Inhalt des Speichers 118 bei jedem Niederdrücken des Daten-Eingabeschalters 39 um Eins erhöht. Der Inhalt des Speichers 118 wird auf die Anzahl eingestellt, die der Therapeut als wünschenswert festlegt, um dem Patienten die Inhalierung eines vorbestimmten minimalen Luftvolumens vorzugeben. Der Therapeut gibt sodann den Speicher-Zugriffsschalter 38 frei und drückt diesen erneut nieder, um Zugriff auf den «Durchsatzmengenwert-Speicher» 106 im Schritt 56 zu gewinnen. Während der Speicher-Zugriffsschalter 38 im niedergedrückten Zustand gehalten wird, wird der Daten-Eingabeschalter 39 nacheinanderfolgend niedergedrückt, wodurch der Inhalt des Speichers 106 so lange fortgeschaltet wird, bis er der Einstellung des Atemstromreglers 26 an dem Atemstrommesser 18 entspricht, was in dem Schritt 58 angezeigt ist. Der Therapeut gibt sodann den Speicher-Zugriffsschalter 38 frei und drückt diesen erneut nieder, wodurch Zugriff auf den «Volumenwert-Speicher» 112 im Schritt 60 gewonnen wird. Bei niedergedrücktem Zugriffsschalter 38 betätigt der Therapeut nacheinanderfolgend den Daten-Eingabeschalter 39. Durch dieses nacheinanderfolgende Niederdrücken des Daten-Eingabeschalters 39 wird der Inhalt der «Volumenwert-Speicher» 112 in Schritten fortgeschaltet, die dem Inhalt des «Durchsatzmengenwert-Speichers» 106 multipliziert mit einer halben Sekunde entsprechen. Wenn somit der Speicher 106 auf 1440 Kubikzentimeter pro Sekunde eingestellt ist, so wird der Inhalt des Speichers 112 in Schritten von 720 Kubikzentimetern bei jedem Niederdrücken des Daten-Eingabeschalters 39 gemäss dem Schritt 62 fortgeschaltet. An dieser Stelle gibt der Therapeut den Speicher-Zugriffsschalter 38 erneut frei und drückt diesen erneut nieder, wodurch Zugriff auf den «Halte-Speicher» 120 gemäss dem Schritt 64 gewonnen wird. Bei niedergedrücktem Speicher-Zugriffsschalter 38 drückt der Therapeut im Schritt 66 nacheinanderfolgend den Daten-Eingabeschalter 39 nieder und erhöht hierdurch den Inhalt des «Halte-Speichers» 120 um eine halbe Sekunde bei jeder aufeinanderfolgenden Niederdrückung des Daten-Eingabeschalters 39. Der Inhalt des «Halte-Speichers» zeigt die Anzahl der Sekunden an, die der Therapeut dem Patienten vorgibt, um den Atem anzuhalten, nachdem er das maximale Luftvolumen entsprechend seiner Fähigkeit eingeatmet hat. Der Inhalt des «Volumenwert-Speichers» 112 gibt das minimale Luftvolumen vor, das der Therapeut dem Patienten zum Inhalieren vorgibt, um den speziellen Inhalierversuch als erfolgreich betrachten zu können.

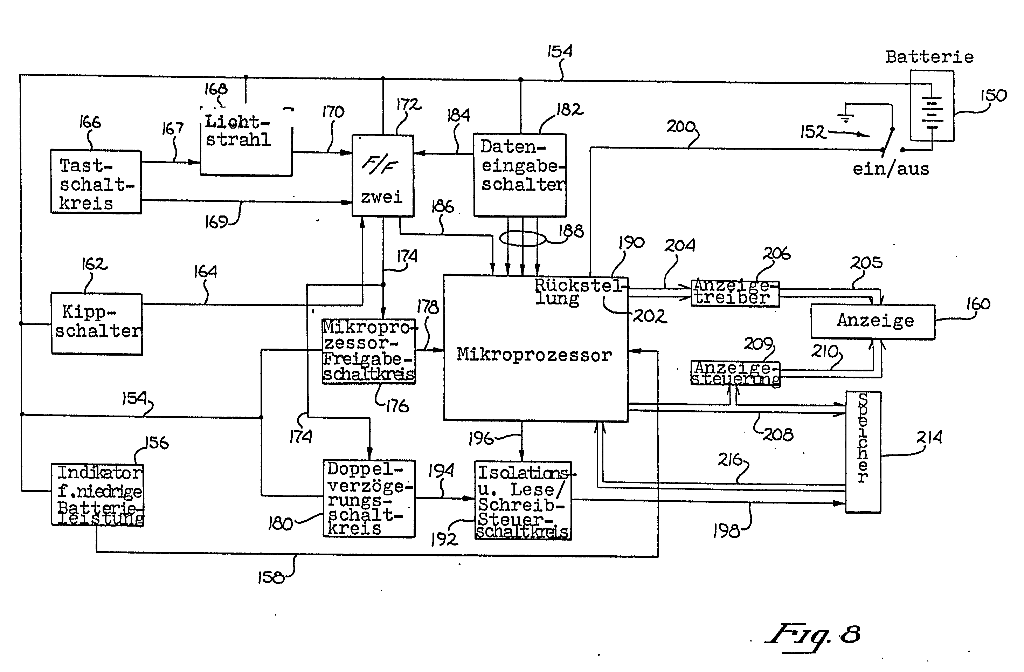

Nachdem der Therapeut den Inhalt der verschiedenen Speicher in der zuvor beschriebenen Weise eingestellt hat, werden der Atemstrommesser 18 und das Zusatzgerät 8 dem Patienten für den Gebrauch überlassen, um das durch den Therapeuten vorgeschriebene Atem-Trainingsprogramm auszuführen. Bei der Verwendung durch den Patienten befindet sich das Gerät im Patienten-Operationsmodus. Dieser Operationsmodus ist graphisch in Fig. 7 veranschaulicht.

Das Gerät wird dem Patienten übergeben, wobei das Zusatzgerät 8 eingeschaltet wird. In diesem Zustand stehen die Lichtquelle 30 und der Fotodetektor 32 miteinander in Verbindung, wobei die Verbindung über den von der Lichtquelle 30 zu dem Detektor 32 übertragenen Lichtstrahl erfolgt. Um das Gerät zu betätigen, muss der Patient in ein Rohr 19 atmen, das an den Atemstrommesser 18 angeschlossen ist. Diese Einatmung veranlasst den Indikatorkörper 24 zum Anstieg innerhalb der Kammer 22 und wenn der Luftstrom durch die Kammer 22 ein vorbestimmtes Minimum erreicht, so erreicht der Indikatorkörper 24 das obere Ende der Kammer 22.

Dem Patienten wird somit eine Einatmung befohlen, um den Indikator gemäss dem Schritt 70 anzuheben. Der Indikator steigt in der Kammer 22 an und unterbricht gemäss dem Schritt 72 den Lichtstrahl. Der Indikatorkörper 24 ist eine Kugel innerhalb der Kammer 22. Wenn der Indikator 24 den Strahl zwischen Lichtquelle 30 und Fotodetektor 32 unterbricht, so wird durch die Unterbrechung des Strahls ein Zeitgeber gestartet, was durch den Schritt 74 veranschaulicht ist, und die Unterbrechung des gleichen Strahles schaltet im Schritt 76 ebenfalls den Mikroprozessor ein.

Im vorliegenden Ausführungsbeispiel ist die Einschaltung des Mikroprozessors nicht buchstäblich als Einschaltung zu verstehen, sondern lediglich eine Veränderung des Zustandes des Mikroprozessors von einem Zustand geringer Leistungsaufnahme zu einem Zustand höherer Leistungsaufnahme, bei welchem der Mikroprozessor vollständig aktiviert ist. Bei der weiteren Erläuterung bedeutet daher die Einschaltung des Mikroprozessors die Umschaltung von einem Zustand niedriger Leistungsaufnahme in einen Zustand hoher Leistungsaufnahme. Nachdem der Zeitgeber 102 gestartet ist und der Mikroprozessor sich im eingeschalteten Zustand befindet, führt der Mikroprozessor eine Zeitintegration des Inhalts des «Durchsatzmengenwert-Speichers» 106 im Schritt 78 aus. Diese Integration ist im wesentlichen durch eine Multiplikation des in den Mikroprozessor eingegebenen Durchflusses mit derjenigen Zeit gegeben, die verstrichen ist und durch den Zeitgeber angezeigt wird. Dieses laufende Ergebnis wird in der linken Anzeigeeinheit 14 dargestellt, und der Inhalt des «Volumenwert-Speichers» wird in der rechten Anzeigeeinheit 16 gemäss dem Schritt 80 dargestellt. Wenn die zeitliche Integration des Durchflusses den Inhalt des «Volumenwert-Speichers» 112 überschreitet, so wird die rechte Anzeigeeinheit 16 veranlasst, das Wort «gut» anstelle des Inhalts des «Volumenwert-Speichers» anzuzeigen. Dies ist in dem Schritt 82 gemäss Fig. 7 veranschaulicht. Diese Anzeige setzt sich so lange fort, wie der Patient in der Lage ist, eine Luftmenge einzuatmen, um den Indikator an dem oberen Ende der Kammer 22 und den Lichtstrahl im unterbrochenen Zustand zu halten.

Wenn der Indikator 24 vom oberen Ende der Kammer 22 herunterfällt, so ist der Lichtstrahl erneut nicht unterbrochen, und der Mikroprozessor erfasst im Schritt 84, dass der Indikator 24 vom oberen Ende der Kammer 22 heruntergefallen ist. Wenn der Indikator 24 einmal das obere Ende der Kammer 22 verlassen hat, so wird die rechte Anzeigeeinheit 16 das Wort «halten» anzeigen, was gemäss dem Schritt 86 das Wort «gut» ersetzt. Die linke Anzeigeeinheit 14 wird das vor dem Fallen der Kugel inhalierte Gesamtvolumen anzeigen. Diese Anzeige des Wortes «halten» setzt sich über einen Zeitraum fort, der dem Inhalt des «Halte-Speichers» 120 entspricht, welcher durch den Therapeuten während des Daten-Eingabemodus eingegeben wurde. Es sei vermerkt, dass, nachdem der Indikator einmal seine Position verlassen hat, das Wort «halten» dargestellt wird und die nacheinanderfolgende Anzeige von errechneten Daten fortgesetzt wird. Sollte der Patient einatmen und den Indikator in einem Versuch anheben, um seine aufgezeichnete Leistung zu verbessern, so wird das Vorhandensein des Indikators so lange nicht erkannt, bis das Gerät zur Überwachung eines erneuten Versuches bereit ist. Während der Zeitdauer, in der das Wort «halten» angezeigt wird, stellt der Mikroprozessor fest, ob das durch den Patienten inhalierte Gesamt-Luftvolumen während der Zeit, in der sich der Indikator 24 an der Spitze der Kammer 22 befindet, den Inhalt des «Volumenwert-Speichers» 112 überschreitet oder nicht. Ist dies der Fall, so erhöht der Mikroprozessor den Inhalt des Speichers «erfolgreiche Atemvorgänge» 116 und zeigt dessen Inhalt in der Anzeigeeinheit 14 zu der gleichen Zeit an, in der der Inhalt des Speichers 118 gemäss dem Schritt 88 in der rechten Anzeigeeinheit 16 erscheint. Auf diese Weise wird der Patient darüber informiert, ob die Anzahl der erfolgreichen Anstrengungen, die er erzielt hat, der Anzahl der erfolgreichen Anstrengungen gleich ist oder nicht, die der Therapeut für einen bestimmten Patienten als geeignet vorgegeben hat. Wenn der Patient während des Schrittes 80 nicht in der Lage war, das vorbestimmte minimale durch den Therapeuten geforderte Luftvolumen einzuatmen, so wird das Wort «gut» nicht, wie zuvor im Schritt 82 beschrieben, angezeigt, und der Mikroprozessor schreitet zu dem Schritt 84 und 86 weiter. Der Mikroprozessor vergleicht sodann im Schritt 90 den Inhalt des «Höchstvolumen-Speichers» 114 mit dem Wert der laufenden Integration des Durchflusses. Wenn die Integration des Durchflusses den Inhalt des Speichers 114 überschreitet, so wird der Inhalt dieses Speichers mit dem Wert des zeitlich integrierten Durchflusses ausgetauscht. Dies ermöglicht es dem Therapeuten, den Mikroprozessor zu einem späteren Zeitpunkt abzufragen und durch Niederdrücken des Speicher-Zugriffsschalters 38 Zugriff auf den Höchstvolumen-Speicher 114 zu nehmen und das während der vorangegangenen Übung durch den Patienten inhalierte maximale Luftvolumen festzustellen.

Der Mikroprozessor schaltet sich sodann automatisch selbst in den Zustand geringer Leistungsaufnahme und wartet die nächste Unterbrechung des Lichtstrahles gemäss dem Schritt 92 ab.

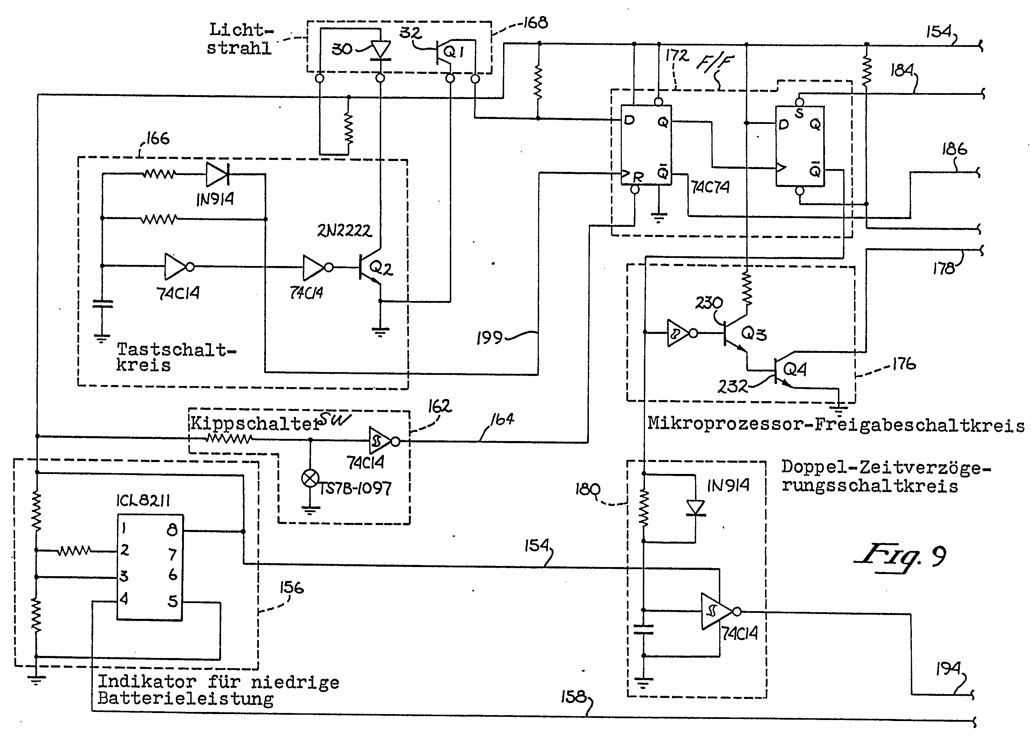

Die Fig. 6 und 7 beschreiben die Operationsfolge während des Daten-Eingabemodus und des Patienten-Operationsmodus des Zusatzgerätes 8. Fig. 8 gibt eine grundlegende funktionelle Beschreibung der verschiedenen Elemente des Zusatzgerätes 8 gemäss der vorliegenden Erfindung.

Das Zusatzgerät 8 wird mit Spannung über eine Batterie 150 versorgt. Ein Batterielade-Netzgerät kann verwendet werden, wenn die Batterieleistung abgenommen hat oder um die Lebensdauer der Batterie zu erweitern, wenn eine Wechselspannungsquelle verfügbar ist. Die Batteriespannung wird durch den Schalter 152 entweder ein- oder ausgeschaltet. Die gemeinsame Spannungsbezugsbasis für die Schaltung gemäss dem Blockdiagramm in Fig. 8 wird durch die Leitung 200 vorgegeben, die das Massepotential führt. Während die Verbindung dieser Leitung zu jedem der verschiedenen Elemente nicht dargestellt ist, ist es jedoch verständlich, dass jedes der verschiedenen Elemente in geeigneter Weise an ein Massepotential entsprechend der Leitung 200 angeschlossen ist. Die Spannung wird den verschiedenen Elementen in geeigneter Weise über eine Leitung 154 zugeführt. Das Blockdiagramm gemäss Fig. 8 ist am leichtesten verständlich, indem mit einer Erläuterung der Betriebsweise des Lichtstrahles 168 begonnen wird. Es sei vermerkt, dass der Lichtstrahl nicht kontinuierlich eingeschaltet ist, sondern dass dies nur während ungefähr 10% der Zeitdauer der Fall ist. Dies wird durch den Tastschaltkreis 166 hervorgerufen, der den Lichtstrahl mit ungefähr 100 Hertz im Pulsbetrieb betreibt. Der Lichtstrahl wird für ungefähr eine Millisekunde 100mal pro Sekunde eingeschaltet. Die verschiedenen Zeitverzögerungen sind jedoch dergestalt, dass die Tastung des Lichtstrahles weder den Zeitgeber noch den Mikroprozessor aktiviert. Nur eine längere Unterbrechung beispielsweise bei Vorhandensein des Indikators 24 verursacht die Unterbrechung des Lichtstrahles in einer Weise, durch die der Flip-Flop-Schaltkreis 172 getriggert wird, welcher seinerseits die Aktivierung des Zeitgebers und des Mikroprozessors hervorruft. Der Tastschaltkreis 166 dient dem pulsierenden Betrieb des Lichtstrahles 168 mittels eines Signales, das über eine Leitung 167 gesendet wird. Der Tastschaltkreis dient ebenfalls als Takt für den Flip-Flop-Schaltkreis 172, wobei ein entsprechendes Signal über die Leitung 169 übertragen wird. Es ist die Funktion des Flip-Flop-Schaltkreises 172, den Zeitgeber und den Mikroprozessor einzuschalten, wenn geeignete logische Zustände vorliegen. Der Mikroprozessor wird eingeschaltet, wenn der Lichtstrahl durch den Indikatorkörper 24 unterbrochen wird und wenn der Speicher-Zugriffsschalter 38 aktiviert ist. Der Mikroprozessor wird abgeschaltet, wenn der Kippschalter 162 das geeignete Signal über die Leitung 164 an das Flip-Flop 172 sendet, welches daraufhin ein logisches Signal erzeugt, das zu der Abschaltung des Mikroprozessors führt. Der Mikroprozessor wird ebenfalls eine vorbestimmte Zeit nach der Ausführung des letzten Anzeigebefehls innerhalb der vorprogrammierten Befehle, die den Betrieb des Mikroprozessors steuern, abgeschaltet. Wenn der geeignete logische Zustand vorliegt, so startet das Flip-Flop 172 den Zeitgeber durch ein über die Leitung 186 gesendetes Signal, oder es sendet ein geeignetes Signal über die Leitung 174 an den Mikroprozessor-Freigabeschaltkreis 176. Dieser Mikroprozessor-Freigabeschaltkreis 176 sendet nach einer geeigneten Zeitverzögerung ein Signal über die Leitung 178, wodurch der Mikroprozessor aktiviert wird.

Diese Verzögerung stellt eine von zwei Verzögerungen dar, die durch den Schaltkreis innerhalb des Doppel-Verzögerungsblockes 180 eingestellt werden. Dieser Doppel-Verzögerungsschaltkreis liefert eine Verzögerung für die Einschaltung des Mikroprozessors und eine weitere Verzögerung, die den Mikroprozessor für eine vorgewählte Zeitdauer nach der Bearbeitung des letzten Anzeigeschrittes der Mikroprozessor-Programmbefehle eingeschaltet hält.

Wenn das Zusatzgerät in dem Daten-Eingabemodus betrieben wird, so bilden die Daten-Eingabeschalter 182 den geeigneten Logikzustand für das Flip-Flop 172, so dass die Betätigung der Daten-Eingabeschalter die Eingabe der gewünschten Daten über die Leitungen 188 in den Mikroprozessor veranlassen. Ein weiteres Eingangssignal wird dem Mikroprozessor über die Leitung 158 von dem Indikator 156 für eine niedrige Batterieleistung zugeführt. Dieser Schaltkreis ist dergestalt, dass er über ein Signal an den Mikroprozessor die Aktivierung der Anzeigeeinrichtung 160 veranlasst, um eine Dezimalstelle in jeder Ziffer der Anzeigeeinheiten 14 und 16 anzuzeigen, wenn die zugeführte Batterieleistung unter einen bestimmten Pegel gefallen ist. Da der Mikroprozessor 190 in zwei Betriebsweisen arbeitet, d.h. mit niedriger und hoher Leistungsaufnahme, können bei der Umschaltung des Mikroprozessors auf hohe Leistungsaufnahme zufällige Signale erzeugt werden, die, falls sie nicht kontrolliert werden, mit dem Inhalt des externen Speichers 214 überlappen. Eine solche Überlappung kann zu einer totalen Zerstörung des Inhalts des Speichers 214 führen. Um eine solche Zerstörung des Speichers zu vermeiden, ist ein Isolations- und Lese/Schreib-Steuerschaltkreis 192 vorgesehen, der zusammen mit dem Doppel-Verzögerungsschaltkreis 180 die zufälligen Signale des Mikroprozessors an eine Überlappung mit dem Inhalt des Speichers 214 hindert. Dieser Isolations- und Lese/Schreib-Steuerschaltkreis 192 steuert den logischen Zustand des Lese/Schreib-Einganges für den externen Speicher 214 durch eine geeignete Steuerung des Logikzustandes auf der Leitung 198. Der externe Speicher 214 wird benutzt, um die verschiedenen Daten zu speichern, die der Therapeut eingibt, während sich der Mikroprozessor in dem Zustand geringer Leistungsaufnahme befindet. Wenn der Lichtstrahl 168 unterbrochen wird, so wird der Mikroprozessor eingeschaltet, und der Inhalt des externen Speichers 214 wird zu dem internen Speicher des Mikroprozessors zur Verwendung bei der Verarbeitung der verschiedenen Signale übertragen. Wenn der Indikator 24 einmal von dem oberen Ende der Kammer 22 herabgefallen ist, so hat der Mikroprozessor die verschiedenen Werte errechnet und in den geeigneten Speichern abgelegt. Die Anzeigeeinrichtung 160, die von der Anzeigesteuerung 209 gesteuert wird, zeigt der Reihe nach in den Anzeigeeinheiten 14 und 16 den Inhalt der verschiedenen Speicher des Mikroprozessors an. Die Spannung für die Anzeige 160 wird über Anzeigetreiber 206 zugeführt, die über Leitungen 204 an den Mikroprozessor und über Leitungen 205 an die verschiedenen Ziffern der Anzeigeeinrichtung 160 angelegt sind. Die Anzeigesteuerung 209 legt fest, welcher Inhalt der verschiedenen Speicher zu einem vorgegebenen Zeitpunkt angezeigt wird. Nachdem der Anzeigezyklus einmal vervollständigt worden ist, stellen die vorprogrammierten Befehle für den Betrieb des Mikroprozessors diesen einschliesslich der Datenspeicher zurück, um die nächste Unterbrechung des Lichtstrahles 168 abzuwarten.

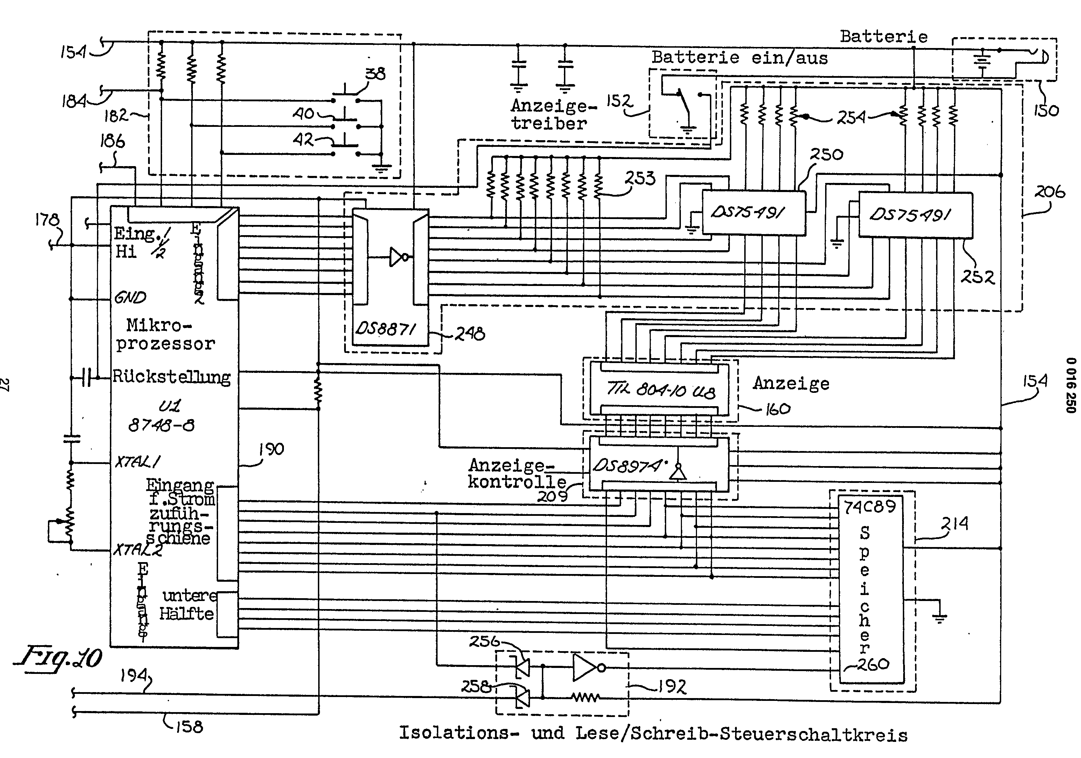

Während die Beschreibung von Fig. 8 ziemlich funktionell erfolgte, ist der Inhalt der verschiedenen Blöcke des Blockdiagramms gemäss Fig. 8 detaillierter in den Fig. 9 und 10 veranschaulicht. Diese beiden Figuren stellen verschiedene Teile des Blockdiagramms gemäss Fig. 8 dar, wobei die verschiedenen Schaltkreiselemente innerhalb jedes Blockes in näheren Einzelheiten dargestellt sind. Beispielsweise ist der Tastschaltkreisblock in Fig. 9 durch den Block 166 näher dargestellt. Die Schaltkreiselemente innerhalb des Blockes 166 in Fig. 9 bilden eine bestimmte Schaltungsanordnung, die die Funktionen des Tastschaltkreises ausführt, wie sie im Zusammenhang mit Fig. 8 erläutert wurden. In gleicher Weise besitzt jeder der Blöcke in Fig. 8 ein geeignetes Gegenstück in Fig. 9 oder in Fig. 10. Die verschiedenen Schaltkreisausführungen gemäss den Fig. 9 und 10 innerhalb der verschiedenen Blöcke sind dergestalt, dass sie die Funktionen der Blöcke ausführen, wie sie unter Bezugnahme auf Fig. 8 erläutert wurden. Beispielsweise entspricht der Block des Anzeigetreibers 206 in Fig. 8 dem Schaltkreis innerhalb des Blockes 206 in Fig. 10. Der Anzeigetreiber 206 umfasst in Wirklichkeit drei verschiedene Treiberschaltkreise 248, 250 und 252 sowie mehrere Strombegrenzungswiderstände 253 und 254. Diese drei Treiber und die zwei Widerstandsanordnungen könnten ebensogut durch einen einzigen Treiber mit geeigneter Leistungsfähigkeit ersetzt werden. Der Mikroprozessor 190 gemäss Fig. 8 ist in Fig. 10 als ein Mikroprozessor des Typs mit der Fabrikationsnummer 8748-8 dargestellt, der von der Firma Intel hergestellt und vertrieben wird.

Eine detailliertere Erläuterung der Wirkungsweise der verschiedenen Komponenten des Zusatzgerätes 8 kann unter Zuhilfenahme der Fig. 9 und 10 erfolgen. Es sei ausdrücklich darauf verwiesen, dass sich die Fig. 9 und 10 einander ergänzen und dass sich die Leitungen auf der äusseren rechten Seite in Fig. 9 auf der äusseren linken Seite in Fig. 10 fortsetzen. Bei der detaillierten Erläuterung der Betriebsweise des Gerätes sei mit dem Tastschaltkreis 166 begonnen.

Der Tastschaltkreis in dem Block 166 gemäss Fig. 9 erzeugt ein Signal, das an der Basis des Transistors Q2 zwischen dem hohen und niedrigen Logikpegel umschaltet. Hierdurch schaltet der Transistor Q2 seinerseits ein und aus, wodurch die Lichtquelle 30 entsprechend ein- und ausgeschaltet wird. Wenn die Energiequelle 30 eingeschaltet ist, so erzeugt sie Energie, die auf den Energiedetektor 32 gemäss Fig. 9 in Form eines Transistors Q1 auftrifft. Das oszillierende Signal des Tastschaltkreises wird ebenfalls ·als Taktsignal für den Flip-Flop-Schaltkreis 172 benutzt. Es ist erkennbar, dass beim Auftreffen des Lichtstrahls auf den Transistor Q1 dieser eingeschaltet wird und der Eingang D des Flip-Flops 17 an Masse angelegt wird. Jedesmal, wenn das Flip-Flop 172 das Taktsignal über die Leitung 199 empfängt, nimmt der Ausgang Q des Flip-Flops den niedrigen Logikpegel ein, und der Ausgang Q besitzt den hohen Logikpegel. Mit dem Ausgang Q auf dem hohen Logikpegel ist der Mikroprozessor-Freigabeschaltkreis 176 und somit der Mikroprozessor ebenfalls abgeschaltet. Bei hohem Logikpegel für das Signal Q ist der Zeitgeber ebenfalls gesperrt. Es ist daher erkennbar, dass, wenn der Indikator 24 nicht zwischen der Lichtquelle 30 und dem Fotodetektor 32 angeordnet ist, der Zeitgeber und der Mikroprozessor sich im ausgeschalteten Zustand befinden. Wenn der Indikator 24 sich zwischen der Lichtquelle 30 und dem Fotodetektor 32 befindet, so trifft der Lichtstrahl nicht länger auf die Basis des Fotodetektors 32 (Q1) auf, und dieser Detektor befindet sich im ausgeschalteten Zustand. Hierdurch tritt ein hoher Logikpegel am Eingang D des Flip-Flops 172 auf und wenn das Flip-Flop in geeigneter Weise durch das Tastsignal auf der Leitung 199 getaktet wird, so nimmt der Ausgang Q den hohen Logikpegel und der Ausgang Q den niedrigen Logikpegel ein. Ein niedriger Logikpegel am Ausgang Q startet den Zeitgeber und schaltet die Transistoren Q3 und Q4 ein, wodurch ein Signal auf der Leitung 178 auftritt, welches den Mikroprozessor einschaltet.

Es ist ebenfalls ersichtlich, dass für den Fall, dass das Zusatzgerät 8 mehr als ein vorgegebenes Mass gegenüber der Vertikalen verschoben ist, der Kippschalter 162 zur Veränderung des geöffneten Zustandes veranlasst wird, wodurch ein Signal mit niedrigem Logikpegel über die Leitung 164 an das Flip-Flop 172 gesendet wird, wodurch der Zeitgeber und der Mikroprozessor in den ausgeschalteten Zustand gebracht werden.

Der Indikatorschaltkreis 156 für geringe Batterieleitung vergleicht den Spannungspegel der Spannungsquelle 150 mit einem vorprogrammierten Referenz-Spannungspegel und für den Fall, dass die Spannungsquelle 150 mit ihrem Wert unter den vorprogrammierten Pegel fällt, wird ein Signal über die Leitung 158 an den Mikroprozessor gesendet, wodurch jede Ziffer der Anzeige 160 auf eine Dezimalstelle gesetzt wird.

Der Doppel-Zeitverzögerungsschaltkreis 180 verzögert die Operation des Mikroprozessor-Freigabeschaltkreises 176 und verhindert zusammen mit dem Trennschaltkreis 192 die Erzeugung von Zufallssignalen durch den Mikroprozessor 190, wodurch Daten in den Speicher 214 zu einem Zeitpunkt eingeschrieben werden könnten, die den Dateninhalt des Speichers zerstören würden. Der Doppel-Zeitverzögerungsschaltkreis 180 liefert eine unterschiedliche Zeitverzögerung für die Einschaltung des Mikroprozessors im Hinblick auf die Zeit, die sich der Mikroprozessor selbst zur Einschaltung vorgibt.

Gemäss Fig. 10 arbeitet der Isolations- und Lese/Schreib-Steuerschaltkreis 192 mit dem Doppel-Zeitverzögerungsschaltkreis 180 in der zuvor erläuterten Weise zusammen. Der Isolations- und Lese/Schreib-Steuerschaltkreis arbeitet im wesentlichen als ein NAND-Gatter mit zwei Eingängen, und der Ausgang dieses Schaltkreises legt den Logikzustand des Lese/Schreib-Steueranschlusses für den Speicher 214 fest. Dieser Schaltkreis 192 ändert die zufälligen Ausgangssignale des Mikroprozessors, die während der Ein- und Ausschaltzeit erzeugt werden, daran, fehlerhafte Daten in den Speicher 214 einzuschreiben. Dieser Schaltkreis hindert im wesentlichen Daten so lange am Einschreiben in den Speicher 214, bis das Signal an beiden Schottky-Dioden 256 und 258 den hohen Logikpegel aufweisen. Nur bei diesem Zustand können Daten in den Speicher 214 eingeschrieben werden. Der Mikroprozessor 190 kann ebenfalls durch Betätigung des Schalters 38 eingeschaltet werden, wodurch die Leitung 184 an Masse gelegt wird und wodurch anderseits das Flip-Flop 172 so beeinflusst wird, dass der Ausgang Q den niedrigen Logikpegel aufweist, wodurch die Transistoren Q3 und Q4 und somit auch der Mikroprozessor eingeschaltet werden. Eine geeignete Betätigung des Daten-Eingabeschalters 39 veranlasst den Mikroprozessor zur Erhöhung des Inhalts eines ausgewählten Speichers, vorausgesetzt, der Schalter 38 wird niedergedrückt. Die Betätigung des Ein/Aus-Schalters 41 veranlasst den Mikroprozessor zur Löschung des Inhalts aller Speicher, während die Betätigung des Löschschalters 40 nur den Inhalt eines ausgewählten Speichers löscht.

Der Mikroprozessor 190 wird gesteuert und betrieben gemäss einer vorprogrammierten Gruppe von Befehlen, und er steuert daraufhin die Anzeigetreiber 206, die Anzeigeeinrichtung 160, die Anzeigesteuerung 209 und den Speicher 214. Eine entsprechende Liste dieser Befehle ist als Appendix A am Ende dieser Beschreibung beigefügt.

Die erwähnte Befehlsgruppe kann für einen Programmierer hilfreich sein, die Wirkungsweise des beschriebenen Gerätes zu verstehen. Eine sehr viel einfachere sichtbare Hilfe ist jedoch in Fig. 11 dargestellt. Mit Ausnahme des Detektors 100 und der Anzeigeeinrichtung 160 stellt Fig. 11 eine Funktionsdarstellung des Mikroprozessors 190 dar. Gemäss Fig. 11 erzeugt der Detektor 100 ein Signal, welches festlegt, ob der Zeitgeber 102 starten oder anhalten soll, und er erzeugt ebenfalls ein Signal, das den Mikroprozessor startet. Das Ausgangssignal des Zeitgebers wird von einem Multiplikationsschaltkreis 104 verwendet, der im wesentlichen eine Zeitintegration des Inhalts des «Durchsatzmengenwert-Speichers» 106 ausführt. Der Inhalt des Speichers 106 ist durch den Therapeuten vor dem Betrieb des Gerätes durch den Patienten eingestellt worden. Durch Multiplikation des Ausganges des Zeitgebers mit dem Inhalt des Speichers 106 wird eine effektive Zeitintegration des Durchflusses erzielt. Am Ausgang des Multiplizierers 104 entsteht somit ein Signal, das dem laufenden Gesamt-Luftvolumen entspricht, das durch den Patienten geatmet wird.

Diese laufende Gesamtmenge des eingeatmeten Luftvolumens wird ferner durch den Mikroprozessor benutzt, um drei Operationen auszuführen. Die erste Operation wird durch den Vergleicher 108 ausgeführt, der das laufende Volumen der tatsächlich eingeatmeten Luft mit dem Inhalt des «Volumenwert-Speichers» 112 vergleicht. Wenn der Vergleich anzeigt, dass die Zeitintegration des Durchflusses den Inhalt des «Volumenwert-Speichers» übersteigt, so wird über die Anzeigeeinrichtung das Wort «gut» angezeigt und der Speicher «erfolgreiche Atemvorgänge» 116 wird um Eins erhöht, um anzuzeigen, dass der Patient erfolgreich ein Luftvolumen inhaliert hat, das dasjenige überschreitet, welches durch den Therapeuten in den «Volumenwert-Speicher» 112 eingegeben wurde. Der Inhalt sowohl des Speichers 112 als auch des Speichers 116 ist auf Abruf zur Anzeige verfügbar.

Die zweite Verwendung der laufenden Gesamtmenge des durch den Patienten eingeatmeten Luftvolumens erfolgt durch den Vergleicher 110. Das laufende Gesamt-Luftvolumen, welches von dem Patienten inhaliert wird, wird mit dem Inhalt des «Höchstvolumen-Speichers» 114 verglichen. Die Befehle des Mikroprozessors steuern den Betrieb dieses Speichers, und der Inhalt dieses Speichers entspricht dem höchsten Luftvolumen, welches der Patient in einer vorgegebenen Reihe von Einatmungen eingeatmet hat. Wenn das durch den Patienten inhalierte Gesamt-Luftvolumen das Höchstvolumen überschreitet, das zuvor von dem Patienten eingeatmet und in dem Speicher 114 gespeichert wurde, so wird der Inhalt des «Höchstvolumen-Speichers» 114 durch das laufende Gesamt-Luftvolumen ersetzt, das von dem Patienten eingeatmet wurde und am Ausgang des Multiplizierers 104 zur Verfügung steht. Andere Speicher, deren Operation durch den Mikroprozessor und seine Befehle gesteuert werden, sind der Speicher «erforderliche Atemvorgänge» 118 und der «Halte-Speicher» 120. Der Inhalt dieser Speicher ist durch den Therapeuten vor dem Betrieb des Gerätes durch den Patienten eingestellt worden. Der Inhalt des «Höchstvolumen-Speichers» 114, des Speichers «erforderliche Atemvorgänge» 118 und des «Halte-Speichers» 120 ist jeweils auf Anforderung zur Anzeige verfügbar.

Die dritte Verwendung des Ausganges des Multiplizierers 104 stellt die direkte Anzeige dar. Die Befehle des Mikroprozessors ermöglichen die Anzeige des laufenden Gesamt-Luftvolumens, das durch den Patienten eingeatmet wird, in der geeigneten Anzeigeeinheit der Anzeigeeinrichtung 160. Nachdem der Patient den minimalen Luftfluss nicht länger aufrechterhalten kann, der erforderlich ist, um den Indikator 24 in dem oberen Bereich der Kammer 22 zu halten, erzeugt der Detektor 100 ein Signal, durch welches der Zeitgeber 102 angehalten wird und der automatische Anzeigezyklus des Mikroprozessors beginnt. Dieser besteht darin, dem Patienten anzuzeigen, dass er seinen Atem für eine vorbestimmte Zeitdauer anzuhalten hat. Nachdem die Zeit verstrichen ist, in der der Patient seinen Atem anzuhalten hat, ändert die Anzeige und zeigt die Anzahl der Anstrengungen an (Inhalt des Speichers «erforderliche Atemvorgänge» 118), die der Therapeut dem Patienten vorgegeben hat. Hierdurch wird die Anzahl der Anstrengungen angezeigt, in der der Patient erfolgreich ein minimales Luftvolumen eingeatmet hat. Nach einer vorbestimmten Zeitdauer verschwindet diese Anzeige aus dem Anzeigefenster, und die Befehle bereiten den Mikroprozessor zur Rückstellung und zur Erwartung des nächsten Startsignals, das durch den Detektor 100 erzeugt wird, vor.

Eine typische Anzeige, die von dem Patienten gesehen wird, ist in Fig. 12 dargestellt. In der Anzeigeeinheit 14 ist das Luftvolumen dargestellt, das durch den Patienten während der Zeit eingeatmet wurde, in der sich der Indikator 24 im oberen Bereich der Kammer 22 befand. In der rechten Anzeigeeinheit ist das Wort «gut» dargestellt, wodurch angezeigt wird, dass das Volumen in der Anzeigeeinheit 14 das durch den Therapeuten programmierte und in den «Volumenwert-Speicher» 112 eingegebene Volumen überschreitet. Die Kombination der linken Anzeigeeinheit 14 und der rechten Anzeigeeinheit 16 ist hier allgemein als Anzeigeeinrichtung 160 bezeichnet.

## Patentansprüche

1. Atemtrainingssystem mit Patientenrückkopplung, bestehend aus einem Atemstrommesser (18) mit einer Grundplatte (20) und einem aufrecht auf dieser angeordneten Geräteteil, welcher eine Ansaugluftmesskammer (22) mit einem darin beweglichen Indikatorkörper (24) aufweist, der sich in eine bestimmte Position bewegt, wenn der Ansaugluftstrom eine bestimmte, mittels eines Atemstromreglers (26) einstellbare Durchflussmenge erreicht oder überschreitet, gekennzeichnet durch ein mit dem Atemstrommesser (18) formschlüssig zu einem Gesamtgerät verbindbares Zusatzgerät (8) mit einem berührungslos durch Aussenden und Empfangen einer Energiestrahlung arbeitenden Signalgeber (30, 32; 100), welcher ein Signal erzeugt, solange sich der Indikatorkörper (24) in der bestimmten Position befindet, mit einer in dem Zusatzgerät (8) angeordneten signalverarbeitenden Auswerteinrichtung und einer am Zusatzgerät (8) vorgesehenen alphanumerischen Anzeigeeinrichtung (160).

2. Atemtrainingssystem nach Anspruch 1 mit einem Atemstrommesser (18), dessen Ansaugluftkammer (22) transparent ist, dadurch gekennzeichnet, dass der Indikatorkörper (24) lichtundurchlässig ist, und der berührungslose Signalgeber (30, 32; 100) aus einer Lichtquelle (30) und einem Fotodetektor (32) besteht, die sich nach Verbinden des Zusatzgerätes (8) mit dem Atemstrommesser (18) zu beiden Seiten der Kammer (22) in einer Lage befinden, in der der Strahlenweg von der Lichtquelle (30) zum Fotodetektor (32) durch den Indikatorkörper (24) unterbrochen wird, wenn dieser sich in der bestimmten Position befindet.

3. Atemtrainingssystem nach Anspruch 1 oder 2, dadurch gekennzeichnet, dass das Zusatzgerät (8) zur formschlüssigen Verbindung mit dem Atemstrommesser (18) eine dem aufrechten Teil des Atemstrommessers (18) komplementäre Ausnehmung (34, 36) aufweist, mittels derer es positionsgenau mit dem Atemstrommesser (18) verbindbar ist.

4. Atemtrainingssystem nach einem der Ansprüche 1–3, dadurch gekennzeichnet, dass die signalverarbeitende Auswerteinrichtung einen zur Eingabe des vom Patienten in einem einzigen Atemzug einzuatmenden vorgegebenen Minimalvolumens bestimmten programmierbaren Volumenwert-Speicher (112) und eine mit dem Signalgeber (30, 32; 100) zusammenarbeitende Volumen-Recheneinrichtung (102–108) aufweist, um durch zeitliche Integration des vom Signalgeber (30, 32; 100) abgegebenen Signals das tatsächlich durch den Patienten in einem Atemzug eingeatmete Luftvolumen zu ermitteln, und dass die Anzeigeeinrichtung (160) eine mit dem Volumenwert-Speicher (112) und der Volumen-Recheneinrichtung (102–108) zusammenarbeitende Anreizanzeige (116–120) einschliesst, die anzeigt, ob das tatsächlich eingeatmete Volumen das vorgegebene Minimalvolumen erreicht bzw. übertroffen hat.

5. Atemtrainingssystem nach Anspruch 4, gekennzeichnet durch einen programmierbaren Durchsatzmengenwert-Speicher (106) zur Eingabe der am Atemstromregler (26) eingestellten Durchsatzmenge, dessen gespeicherte Daten zur Ermittlung des tatsächlich eingeatmeten Volumens von der Volumen-Recheneinrichtung (102–108) verarbeitet werden.

6. Atemtrainingssystem nach Anspruch 5, gekennzeichnet durch einen Speicher (118), «erforderliche Atemvorgänge», für die Eingabe der Anzahl der vom Patienten zu unternehmenden Atemvorgänge, bei denen jeweils das tatsächlich eingeatmete Luftvolumen das vorgegebene Minimalvolumen übersteigen soll, einen Speicher (116), «erfolgreiche Atemvorgänge», der mit der Volumen-Recheneinrichtung (102–108) zusammenarbeitet, um die tatsächliche Anzahl der Atemvorgänge zu zählen, bei denen das vorgegebene Minimalvolumen überschritten wird, und als Teil der Anzeigeeinrichtung (160) eine Anzeigeeinheit (14) für die Anzeige der Speicherinhalte der Speicher «erforderliche Atemvorgänge» (118) und «erfolgreiche Atemvorgänge» (116).

7. Atemtrainingssystem nach einem der Ansprüche 4–6, bei dem sich der Indikatorkörper (24) aufgrund der Atmung des Patienten bewegt, gekennzeichnet durch: einen programmierbaren «Halte-Speicher» (120) für die Eingabe einer vorgegebenen Zeitdauer, für die der Patient seinen Atem nach jeder Einatmung anhalten soll, als Funktion der Anzeigeeinrichtung (160) eine Haltezeit-Ausgabe, die durch die Volumen-Recheneinrichtung (102–108) ausgelöst wird, zur Anzeige der im Halte-Speicher (120) gespeicherten Haltezeit, wobei die Anzeige ausgelöst wird, wenn die Volumen-Recheneinrichtung (102–108) ein Signal erhält, dass sich der Indikatorkörper (24) nicht mehr in seiner bestimmten Position befindet.

8. Atemtrainingssystem nach einem der Ansprüche 4–7, gekennzeichnet durch einen Höchstvolumen-Speicher (114), der mit der Volumen-Recheneinrichtung (102–104; 110) zusammenarbeitet, um den höchsten Wert des tatsächlich eingeatmeten Luftvolumens festzuhalten, das durch den Patienten in irgendeiner Folge von Atemzügen, deren Volumen durch das Gerät gemessen wird, eingeatmet wird, und einen am Zusatzgerät (8) vorgesehenen Schalter (162) zum Auslösen der Anzeige des Inhalts des Höchstvolumen-Speichers (114) in der Anzeigeeinrichtung (160).

9. Atemtrainingssystem nach einem der Ansprüche 4–8, dadurch gekennzeichnet, dass die einzelnen Speicher, die elektrischen Schaltmittel, die Volumen-Recheneinrichtung (102–108), die Zähleinrichtungen und die Ausleseeinrichtungen alle durch einen Mikroprozessor (190) und einen zentralen Speicher (214) verwirklicht sind, wobei der Mikroprozessor (190) an die Schalter (38–41), den Fotodetektor (32) und die alphanumerische Anzeigeeinrichtung (160) angeschlossen ist und durch ein in dem zentralen Speicher (214) gespeichertes Programm gesteuert wird.

## Claims

1. A respiratory exercising system with patient feedback comprising a spirometer (18) with a base (20) and an apparatus portion mounted upright upon said base, said portion comprising an aspiration air metering chamber (22) with an indicator (24) movable therein which moves into a certain position when the aspiration flow rate equals or exceeds a predetermined flow rate settable by means of a respiration flow regulator control (26), characterized by a supplementary device (8) positively lockable with the spirometer (18) to form a combined apparatus, said supplementary device having a signal transmitter (30, 32; 100) operating at no contact by emitting and receiving an energy radiation generating a signal while the indicator (24) is in said certain position, further having a signal processing evaluation unit being arranged within said supplementary device (8), and an alphanumerical display means (160) provided at the supplementary device (8).

2. The respiratory exercising system according to claim 1 having a spirometer (18), the aspiration air chamber (22) of which is transparent, characterized in that the indicator (24) is impervious to light and the noncontact signal transmitter (30, 32; 100) consists of a light source (30) and a photodetector (32) which after connection of said supplementary device (8) to said spirometer (18) are located at opposite sides of said chamber (22) in a position in which the radiation path from said light source (30) to said photodetector (32) is interrupted by said indicator (24) when the latter is in that certain position.

3. The respiratory exercising system according to claim 1 or 2, characterized in that said supplementary device (8) for positive engagement with said spirometer (18) comprises a channel (34, 36) complementary to the upright portion of said spirometer (18), and by which it is connectable in exact position with said spirometer (18).

4. The respiratory exercising system according to one of claims 1–3, characterized in that the signal processing evaluation unit comprises a programmable volume value register (112) for setting the desired predetermined minimum volume to be breathed by the patient in a single breath, and volume calculating means (102–108) cooperating with said signal transmitter (30, 32; 100) for determination by integration overtime of the signal emitted by said signal transmitter (30, 32; 100) of the actual air volume breathed by the patient in one breath, and that the display means (160) includes an incentive display (116–120) cooperating with said volume value register (112) and said volume calculation means (102–108) which indicates, whether the actually inhaled volume has equaled or, respectively exceeded the predetermined minimum volume.

5. The respiratory exercising system according to claim 4, characterized by a programmable flow rate register (106) for entering the flow rate set at the respiration flow regulator control (26), the stored data of which are used in the determination of the actually respirated volume by said volume calculating means (102–108).

6. The respiratory exercising system according to claim 5, characterized by a register (118) «required efforts», for storing the number of breaths to be made by the patient, where respectively the actually breathed air volume is to exceed the predetermined minimum volume, a register (116) «successful breaths» which cooperates with said volume calculation means (102–108) for counting the actual number of breaths where the predetermined minimum volume is exceeded, and as part of the display (160) a display unit (14) for the display of the storage contents of said registers «required efforts» (118) and «successful breaths» (116).

7. The respiratory exercising system according to one of claims 4–6 where the indicator (24) moves because of the patient's respiration, characterized by: a programable «hold register» (120) for the input of a predetermined duration of time for which the patient shall hold his breath after each inhalation, as function of said display (160) a hold-time output being caused by the volume calculation means (102–108) for display of the hold-time stored in said hold register (120), whereby the display is caused when the volume calculation means (102–108) receives a signal that the indicator (24) is no longer in its certain position.

8. The respiratory exercising system according to one of claims 4–7, characterized by a highest-volume register means (114) cooperating with said volume calculation means (104; 110) for storing the highest value of actual minimum volume of air inhaled by the patient in any series of breaths, the volume of which is measured by said device, and a display switch means (162) situated at said supplementary device (8) for causing the display of the contents of the highest volume register means (114) in the display (160).

9. The respiratory exercising system according to one of claims 4–8, characterized in that said individual registers, said electrical switch means, said volume calculation means (102–108), said counter means and said readout means all are implemented by a microprocessor (190) and a central memory (214) whereby said microprocessor (190) is electrically interconnected to said switches (38–41), the photodetector (32) and the alphanumerical display (160) and is driven by a program stored in said central memory (214).

## Revendications

1. Système d'entraînement respiratoire avec couplage réactif avec le patient, comprenant un spiromètre (18) avec une plaque de base (20) et un élément disposé verticalement sur celle-ci qui présente une chambre de mesure de l'air d'aspiration (22) avec un corps indicateur (24) mobile dans celle-ci qui se déplace dans une position déterminée lorsque le courant d'air d'aspiration atteint ou dépasse un débit déterminé ajustable au moyen d'un régulateur de débit respiratoire (26), caractérisé par un appareil additionnel (8) raccordable mécaniquement à un appareil principal avec le spiromètre (18), avec un générateur de signaux (30, 32, 100), fonctionnant sans contact par émission et réception d'un rayonnement d'énergie, qui produit un signal tant que le corps indicateur (24) se trouve dans la position déterminée, avec un dispositif d'exploitation et de traitement des signaux disposé dans l'appareil additionnel (8) et un dispositif d'affichage alphanumérique (160) monté dans l'appareil additionnel (8).

2. Système d'entraînement respiratoire selon la revendication 1, avec un spiromètre (18) dont la chambre de mesure de l'air d'aspiration (22) est transparente, caractérisé en ce que le corps indicateur (24) est opaque à la lumière et en ce que le générateur de signaux sans contacts (30, 32; 100) est constitué d'une source de lumière (30) et d'un photodétecteur (32) qui, après raccordement de l'appareil additionnel (8) au spiromètre (18) sur les deux côtés de la chambre (22), se trouvent dans une position telle que le trajet du rayonnement de la source de lumière (30) au photodétecteur (32) est interrompu par le corps indicateur (24) lorsque celui-ci se trouve dans la position déterminée.

3. Système d'entraînement respiratoire selon la revendication 1 ou 2, caractérisé en ce que, pour son raccordement mécanique avec le spiromètre (18), l'appareil additionnel (8) présente un évidement (34, 36) complémentaire de la partie verticale du spiromètre (18) au moyen duquel il peut être raccordé dans une position précise au spiromètre (18).

4. Système d'entraînement respiratoire selon les revendications 1–3, caractérisé en ce que le dispositif d'exploitation et de traitement des signaux comporte une mémoire programmable de valeur du volume (112) destinée à l'introduction du volume minimal prédéterminé devant être inspiré par le patient en une seule inspiration et un dispositif de calcul du volume (102–108) coopérant avec le générateur de signaux (30, 32; 100) pour déterminer, par intégration dans le temps, des signaux produits par le générateur de signaux (30, 32; 100), le volume respiratoire réellement inspiré par le patient en une seule inspiration, et en ce que le dispositif d'affichage (160) comprend un affichage à excitation (116–120), coopérant avec la mémoire de valeur du volume (112) et le dispositif de calcul du volume (102–108), qui indique si le volume effectivement inspiré a atteint ou dépassé le volume minimal prédéterminé.

5. Système d'entraînement respiratoire selon la revendication 4, caractérisé par une mémoire programmable de valeur de la capacité (106) pour l'introduction de la capacité ajustée à l'aide du régulateur de débit respiratoire (26), les données mises en mémoire dans celle-ci étant traitées par le dispositif de calcul du volume (102–108) pour la détermination du volume effectivement inspiré.

6. Système d'entraînement respiratoire selon la revendication 5, caractérisé par une mémoire (118), «cycles respiratoires nécessaires», pour la mise en mémoire du nombre de cycles respiratoires devant être effectués par le patient au cours desquels le volume d'air effectivement inspiré doit dépasser le volume minimal prédéterminé, une mémoire (116) «cycles respiratoires couronnés de succès» qui coopère avec le dispositif de calcul du volume (102–108) pour compter le nombre effectif de cycles respiratoires lors desquels le volume minimal prédéterminé est dépassé, et, en tant que partie du dispositif d'affichage (160), une unité d'affichage (14) pour l'affichage des contenus des mémoires «cycles respiratoires nécessaires» (118) et «cycles respiratoires couronnés de succès» (116).

7. Système d'entraînement respiratoire selon l'une des revendications 4–6, dans lequel le corps indicateur (24) se déplace du fait de la respiration du patient, caractérisé par: une «mémoire de retenue» programmable (120) pour la mise en mémoire d'un temps prédéterminé pendant lequel le patient doit retenir sa respiration après chaque inspiration, et, en tant que fonction du dispositif d'affichage (160), par une visualisation du temps de retenue qui est déclenchée par le dispositif de calcul du volume (102–108), pour l'affichage du temps de retenue mémorisé dans la mémoire de temps de retenue (120), l'affichage étant déclenché si le dispositif de calcul du volume (102–108) reçoit un signal indiquant que le corps indicateur (24) ne se trouve plus dans sa position déterminée.

8. Système d'entraînement respiratoire selon l'une des revendications 4 à 7, caractérisé par une mémoire de volume le plus élevé (114) coopérant avec le dispositif de calcul du volume (102–104; 110) pour déterminer la valeur la plus élevée du volume d'air qui est inspiré par le patient lors d'une succession quelconque d'inspirations dont le volume a été mesuré par l'appareil, et par un commutateur (162) monté sur l'appareil additionnel (8) pour déclencher l'affichage du contenu de la mémoire de volume le plus élevé (114) par le dispositif d'affichage (160).

9. Système d'entraînement à la respiration selon l'une des revendications 4 à 8, caractérisé en ce que les diverses mémoires, les moyens de commutation électriques, le dispositif de calcul du volume (102–108), les dispositifs de comptage et les dispositifs de lecture sont tous réalisés au moyen d'un microprocesseur (190) et d'une mémoire centrale (214), le microprocesseur (190) étant relié au commutateur (38–41), au photodétecteur (32) et au dispositif d'affichage alphanumérique (160) et en ce qu'il est commandé par un programme stocké dans la mémoire centrale (214).

*Fig.1*

*Fig.2*

*Fig. 3*

*Fig. 4*

*Fig. 5*

Dateneingabe-Modus

50 ┌─────────────────────────┐
│ Spannungsschalter       │
│      betätigen          │
└─────────────────────────┘

52 ┌─────────────────────────┐
│ Zugriff auf den Speicher │
│ 118 "erforderliche      │
│ Atemvorgänge"           │
└─────────────────────────┘

54 ┌─────────────────────────┐
│ Fortschaltung der In-   │
│ halte des Speichers 118 │
│ "erforderliche Atemvorg." │
└─────────────────────────┘

56 ┌─────────────────────────┐
│ Zugriff auf den "Durch- │
│ satzmengenwert-Speicher" │
│ 106                     │
└─────────────────────────┘

58 ┌───────────────────────────────┐
│ Fortschaltung der Inhalte des │
│ "Durchsatzmengenwert-Speichers │
│ bis die gewünschte Durchsatz-  │
│ mengeneinstellung erreicht     │
│ ist.                          │
└───────────────────────────────┘

60 ┌─────────────────────────┐
│ Zugriff auf den "Volu-  │
│ menwertspeicher" 112    │
└─────────────────────────┘

62 ┌─────────────────────────┐
│ Fortschaltung der In-   │
│ halte des "Volumenwert- │
│ speichers"              │
└─────────────────────────┘

64 ┌─────────────────────────┐
│ Zugriff auf den "Halte- │
│ speicher"               │
└─────────────────────────┘

66 ┌─────────────────────────┐
│ Fortschaltung der In-   │
│ halte des Haltespeichers │
└─────────────────────────┘

*Fig. 6*

19

Patienten-Operationsmodus

*Fig. 7*

```
┌─────────────────────────┐
│ inhaliere, um die       │ ╱70
│ Kugel anzuheben         │
└─────────────────────────┘
            │
            ▼
┌─────────────────────────┐
│ erfasse den unter-      │ ╱72
│ brochenen Lichstrahl    │
└─────────────────────────┘
      │              │
      ▼              ▼
┌──────────┐   ┌────────────────────┐
│ betätige │╱74│ schalte den Mikro-  │ ╱76
│ den Zeit-│   │ prozessor ein      │
│ geber    │   └────────────────────┘
└──────────┘              │
      │                   ▼
      ▼ 78
┌──────────────┐  ┌──────────────────────────┐
│ integriere den│ │ stelle das gesamte inhalierte│ ╱80
│ Durchfluß über│─▶│ Volumen und die Inhalte des  │
│ die Zeit     │  │ Volumenwertspeichers dar     │
└──────────────┘  └──────────────────────────┘
      │                   │
      │                   ▼
      │        ┌─────────────────────────────────┐
      │        │ Wenn das gesamte Volumen größer ist als│ ╱82
      │        │ die Inhalte des Volumenwertspeichers,  │
      │        │ so stelle "Gut" dar anstatt die Inhalte│
      │        │ des Volumenwertspeichers               │
      │        └─────────────────────────────────┘
      │                   │
      ▼ 84        86       ▼
┌──────────────┐  ┌─────────────────────────────────┐
│ erfasse den  │  │ stelle "anhalten" dar anstatt des Wor-│
│ Lichtstrahl, │─▶│ tes "gut", und zwar für eine Zeit, die│
│ die Kugel fiel│ │ den Inhalten des Haltespeichers ent-  │
│ herab        │  │ spricht,stelle das Gesamtvolumen dar  │
└──────────────┘  └─────────────────────────────────┘
      │                   │
      │        88          ▼
      │        ┌─────────────────────────────────┐
      │        │ steigere und stelle die Inhalte des│
      │        │ "erfolgreiche Atemvorgängespeichers│
      │        │ 116 dar, wenn "gut" dargestellt wird│
      │        │ und stelle die Inhalte des Speichers│
      │        │ "erforderliche Atemvorgänge" dar    │
      │        └─────────────────────────────────┘
      │        90          ▼
      │        ┌─────────────────────────────────┐
      │        │ ersetze die Inhalte des Speichers│
      │        │ "erfolgreiche Atemvorgänge" durch die│
      │        │ Inhalte des "Höchstvolumenspeichers"│
      │        │ und d.Speichers "erfolgr.Atemvorg." │
      │        └─────────────────────────────────┘
      │        92          ▼
      │        ┌─────────────────────────────────┐
      │        │ erniedrige die Spannung am Mikropro-│
      │        │ zessor, warte auf die nächste Unter-│
      │        │ brechung des Lichtstrahls           │
      │        └─────────────────────────────────┘
      │                   │
      ▼                   │
     ⊗ ◀──────────────────┘
```

Fig. 8

Fig. 9

Fig. 10

Isolations- und Lese/Schreib-Steuerschaltkreis

Fotodetekt. ⟋ 100

Start/Stop

SET

Zeit-geber ⟋ 102

Durchsatz-mengenwert-speicher ⟋ 106

Multiplika-tor ⟋ 104

Volumen

SET 112

108

110

Höchst-volumen-speicher 114

Volumen-wertspei-cher

Komparator

Komparator

116

Speicher "erfolgrei-che Atem-vorgänge"

Speicher erforderl. Atemvorg.

SET

Anzeigeeinrichtung

118

SET

Halte-speicher

160

120

122 stelle zurück und warte auf d. nächsten Start

*Fig. 11*

14

16

160

*Fig. 12*